# EUROPEAN PATENT APPLICATION

(11) **EP 4 570 309 A2**
(43) Date of publication of application: **18.06.2025**
(21) Application number: 25167701.9
(22) Date of filing: 07.12.2020
(51) Int. Cl.: A61N 1/40

(54) **APPARATUS FOR DELIVERY OF A TISSUE REGENERATIVE MULTI-DRUG COCKTAIL**

(30) Priority: 06.12.2019 US 201962944707 P
(62) Divisional of application: 20895644.1
(71) Applicant: Trustees of Tufts College, Medford, MA 02155 (US)
(72) Inventor: LEVIN, Michael, Beverly, MA (US); KAPLAN, David L., Concord, MA (US); MURUGAN, Nirosha J., Medford, MA (US)
(74) Representative: Fleuchaus & Gallo Partnerschaft mbB

(57) **Abstract**

Disclosed are apparatus, compositions, and methods for promoting regeneration of tissue on a subject such as a wounded, damaged, or injured appendage, or within a subject such as a wounded, damaged, or injured organ. The disclosed apparatus, composition, and methods include or utilize wearable sleeves and regenerative compositions.

## Description

### CROSS-REFERENCE TO RELATED APPLICATIONS

This application claims the benefit of priority to U.S. Provisional Application Ser. No. 62/944,707 filed on December 6, 2019, the entire contents of which are incorporated by reference herein.

### STATEMENT REGARDING FEDERALLY SPONSORED RESEARCH OR DEVELOPMENT

This invention was made with government support under grant AR055993 awarded by the National Institutes of Health. The government has certain rights in the invention.

### FIELD OF THE INVENTION

The field of the invention relates to regenerative medicine. More specifically, the present disclosure relates to apparatus, compositions, and methods for promoting regeneration of tissue on a subject or within a subject in need thereof. The disclosed apparatus include wearable sleeves and the disclosed compositions include regenerative compositions

### BACKGROUND

The prevalence of human limb loss in the United States alone is expected to increase significantly over the next thirty years, affecting 3.6 million individuals per year by 2050 (Ziegler-Graham *et al.,* 2008), leaving diabetics, veterans of war), trauma survivors (, and those suffering from peripheral artery disease (with limited options in the event of an amputation.

Prior efforts have attempted to induce limb regeneration, including the use of electrical stimulation (Borgens (1982) Science 217, 747-750; Leppik et al. (2015) Sci. Rep., 5, 18353; Smith, (1981) Bioelectrochem. and Bioenergetics, 8(6), 661-670), tissue-guiding biomaterials (Suckow et al. (1999) J. of Invest. Surg., 12(5), 277-287), progenitor cell transplantation (Lin et al., (2013) Dev. Cell, 24(1), 41-51), and the regulation of key molecular pathways (Kawakami et al., (2006) Genes & Dev., 20(23), 3232-3237; Yokoyama et al., (2001) Dev. Biology, 233(1), 72-79). However, they have had limited success in restoring significant growth and patterning of new limbs.

Tissue regeneration involves a cascade of biological events that combine fully to rebuild an excision or appendage that was lost during trauma or amputation. There is a distinct difference between a typical wound healing response and a regenerative response. These two processes, while similar in many embodiments, result in completely different end products. During the course of normal wound healing, many complex biological structures, such as sweat glands, ducts and hair follicles, cannot be rebuilt since the biological machinery to do so is not available. In a typical adult mammalian skin wound, these structures are not regenerated since development of these tissues and organs involve highly specific physiological processes. In addition, normal wound closure and scar formation does not provide an adequate environment for these structures to regenerate. Epimorphic regeneration, on the other hand, is the process during which all original structures are replaced with replications of the originals.

While some complex animals, such as axolotls, natively regenerate limbs, eyes, and other whole organs throughout their, mammals generally exhibit limited regeneration and do not generally exhibit such plasticity and transdifferentiation capacity as do urodeles. Anurans that have matured towards adolescent stages can regenerate their amputated or injured limbs when exposed to regeneration inducers delivered through slow release beads implanted in the amputated tissues. Tissue progenitor cells for induction of regeneration processes may also have use; larval limb progenitor cells have been shown to activate the same Wnt, Shh signaling to promote patterning.

However, it is unknown how well fully non-regenerative, strongly post-metamorphic (adult) *Xenopus* can act as useful animal models for regeneration, as adult frogs fail to regenerate their hindlimbs upon amputation, instead generating featureless cartilaginous spikes (Suzuki et al., (2006) Sci. World J., 6:26-37).

Regenerative sleeves have been used for tissue regeneration of wounded tissue. Conventional sleeves include a septum that allows a needle to repeatedly exchange media within the wound space, a liquid reservoir that keeps the wound moist, and a liquid tight seal that prevents liquid from leaving the sleeve. However, the conventional sleeve is subject to several limitations. In some instances, the liquid tight seal is too tight, and may lead to tissue necrosis. The replacement of media via the needle may directly affect the wound bed.

Additionally, conventional tissue regeneration devices are typically attached to the subject utilizing glue, which is not favorable for long term treatments. Using glue to attach regenerative devices has two primary disadvantages. Glue provides a lack of flexibility of adjustments to the device post attachment. In addition, handling complications exist during surgery and device.

Therefore, improved apparatus and regenerative compositions are desirable and needed.

### SUMMARY

Disclosed are apparatus, compositions, and methods for promoting regeneration of tissue such as a wounded, damaged, or injured site present on an appendage of a live subject, or within a subject such as a wounded, damaged, or injured site present on or within an organ. The disclosed apparatus, compositions, and methods include or utilize wearable sleeves and regenerative compositions.

In one aspect, the disclosure provides therapeutic compositions for promoting tissue. In some embodiments, the regenerative compositions may include multiple components. In certain embodiments, the disclosed regenerative compositions are utilized in the disclosed apparatus as therapeutic compositions, for example, which are present in the material of the inner sleeve that contacts the wounded, damaged, or injured tissue.

In one aspect, the therapeutic composition comprises a growth factor, an inhibitor of prolyl hydroxylase domain (PHD) enzyme, vitamin A or a derivative thereof, and a lipid mediator.

In particular embodiments, the therapeutic composition comprises a growth factor selected from brain-derived neurotrophic factor (BDNF), nerve growth factor (NGF), neurotrophin-3 (NT-3), neurotrophin-4 (NT-4), glial-derived neurotrophic factor (GDNF), ciliary neurotrophic factor (CNTF), and leukemia inhibitor factor (LIF), and a combination thereof. In particular embodiments, the therapeutic composition comprises a nerve growth factor. In particular embodiments, the therapeutic composition comprises brain-derived neurotrophic factor (BDNF).

In some embodiments, the therapeutic composition comprises an inhibitor of PHD enzyme selected from the group consisting of 4,4α-dihydro-4-oxo-1,10-phenanthroline-3-carboxylic acid (1,4-DPCA), N-[(1,3-dicyclohexylhexahydro-2,4,6-trioxo-5-pyrimidinyl)carbonyl]-glycine, 6-Amino-1,3-dimethyl-5-[(2-pyridinylthio)acetyl]-2,4(1H,3H)-pyrimidinedione, 6-Amino-1,3-dimethyl-5-[[2-(2-pyridinyl)-4-quinolinyl]carbonyl]-2,4(1H,3H)-pyrimidinedione, N-[(4-hydroxy-1-methyl-7-phenoxy-3-isoquinolinyl)carbonyl]-glycine, iron chelators, and a combination thereof. In particular embodiments, the PHD inhibitor is 4,4α-dihydro-4-oxo-1,10-phenanthroline-3-carboxylic acid (1,4-DPCA). In particular embodiments, the therapeutic composition comprises 4,4α-dihydro-4-oxo-1,10-phenanthroline-3-carboxylic acid (1,4-DPCA).

In some embodiments, the therapeutic composition comprises a derivative of vitamin A selected from the group consisting of retinoic acid, retinol, retinyl carboxylates, tretinoin, tazarotene, and a combination thereof.

In some embodiments, the therapeutic composition comprises a lipid mediator selected from the group consisting of a resolvin, a metabolic product of omega-3 fatty acids, a derivative of eicosapentaenoic acid a derivative of docosahexaenoic acid, and a combination thereof. In certain embodiments, the lipid mediator is a resolvin. In particular embodiments, the therapeutic composition comprises a resolvin selected from the group consisting of resolvin 5, interleukin 6 (IL-6), interleukin 4 (IL-4), tumor necrosis factor-alpha (TNF-alpha), nuclear factor kappa-light-chain-enhancer of activated B cells (NF-kB), and a combination thereof.

In some embodiments, the therapeutic composition the therapeutic composition comprises an agent that functions in proximo-distal positional information, wherein the agent is selected from the group consisting of bone morphogenetic protein 9 (BMP9), nodal growth differentiation factor, activin, transforming growth factor-beta (TGF-β), fibroblast growth factor 8 (FGF8), and a combination thereof.

In some embodiments, the therapeutic composition comprises a peptide or protein hormone. In some embodiments, the therapeutic composition comprises a peptide hormone selected from the group consisting of growth hormone (GH), insulin-like growth factor-1 (IGF-1), transforming growth factor-beta-1 (TGFβ-1), epidermal growth factor (EGF), Granulocyte-colony stimulating factor (G-CSF), fibroblast growth factor FGF, and a combination thereof.

In some embodiments, the growth factor is present at a dose from 0.1 µg/ml to 1 µg/ml within the therapeutic composition. In some embodiments, the PHD inhibitor is present at a dose from 0.004 µg/ml to 0.024 µg/ml within the therapeutic composition. In some embodiments, the vitamin A or derivative thereof is present at a dose from 0.03 µg/ml to 0.27 µg/ml within the therapeutic composition. In some embodiments, the lipid mediator is present at a dose from 0.006 µg/ml to 0.054 µg/ml within the therapeutic composition. In some embodiments, the peptide or protein hormone is present at a dose from 0.1 µg/ml to 1.0 µg/ml within the therapeutic composition.

In another aspect, the present disclosure provides a method of promoting tissue regeneration in a mammal, where the method includes administering the provided therapeutic compositions to a mammal in an amount sufficient to promote tissue regeneration in the mammal.

In yet another aspect, the present disclosure provides a use of the therapeutic composition for stimulation of regeneration of tissue in a mammal in need thereof.

In another aspect, the disclosed apparatus includes an outer sleeve having a tissue receiving and/or inserting end, a pressing member receiving end opposite the tissue receiving end, and an internal chamber configured to receive and/or contain the tissue. The apparatus further includes an inner sleeve disposed within the outer sleeve, the inner sleeve having an end for receiving the wounded, damaged, or injured tissue or appendage, an engagement receiving end for engaging a pressing member, and an internal chamber configured to receive and/or contain the wounded, damaged, or injured tissue. The pressing member is configured to extend into the internal chamber of the outer sleeve through the pressing member receiving end and bias the engagement receiving end of the inner sleeve towards the tissue such that at least a portion of the wounded, damaged, or injured tissue of the subject is placed in contact with a portion of the internal chamber of the inner sleeve. The inner sleeve may comprise and/or may be formed from a polymeric material, including but not limited to a silk hydrogel material. The apparatus further includes a first end cap engageable with the tissue receiving end of the outer sleeve and comprising an opening configured to receive the tissue and maintain the tissue in the device, and a second end cap engageable with the pressing member receiving end of the outer sleeve.

In some embodiments, the tissue is part of an appendage or an organ.

In some embodiments, the apparatus includes a first threaded adapter disposable within the tissue receiving end of the outer sleeve for selectively coupling the first end cap engageable with the tissue receiving end to the outer sleeve. In some embodiments, the first end cap includes grooves configured to receive threads of the first threaded adapter.

In some embodiments, the apparatus includes a second threaded adapter disposable within the pressing member receiving end of the outer sleeve for selectively coupling the second end cap engageable with the pressing member to the outer sleeve. In some embodiments, the second end cap includes grooves configured to receive threads of the second threaded adapter.

In certain embodiments, the pressing member includes a seat portion configured to receive the engagement receiving end of the inner sleeve, or includes an engagement end configured to rest on a seat portion of the second end cap.

In some embodiments, the engagement receiving end of the inner sleeve includes porous filter medium that sealingly encloses the internal chamber on the engagement receiving end. In some embodiments, the porous filter medium is a synthetic or polymeric membrane. In some embodiments, the apparatus includes a compressible member positioned between the porous filter media and the pressing member. In some embodiments, the compressible member comprises cotton or an encapsulated gel.

In some embodiments, the inner sleeve comprises a protein or polymeric matrix that at least partially fills the internal chamber of the inner sleeve. In some embodiments, the protein or polymeric matrix comprises a three-dimensional porous scaffold. In some embodiments, the porous scaffold may include pores that form a directional pattern. In some embodiments, the porous scaffold includes aligned pores that form substantially aligned channels. The aligned channels in the protein or polymeric matrix may be arranged parallel with the longitudinal axis of the inner sleeve. In some embodiments, the protein or polymeric matrix is selected from silk fibroin, collagen, or combinations thereof.

In some embodiments, the apparatus includes an electrical stimulation device including an anode and cathode, the anode and the cathode configured to be electrically connected to corresponding terminals of a power source, and a portion of the cathode being disposed in the inner sleeve.

In another aspect, the disclosed apparatus includes a pressing member that is moveable in response to growth at the site of the wounded, damaged, or injured tissue, for example, in axial direction towards and/or away from the wounded, damaged, or injured tissue. In some embodiments, the pressing member biases the engagement receiving end of the inner sleeve towards the tissue such that at least a portion of the wounded, damaged, or injured tissue of the subject is placed in contact with a portion of the internal chamber of the inner sleeve, and as tissue regenerates and/or the wounded, damaged, or injured tissue heals, the regenerated tissue and/or healed tissue move the pressing member axially away from the wound, for direct or indirect contact with the engagement receiving end.

In some embodiments, the pressing member is elastic or includes an elastic member that compresses in response to growing tissue. The elastic member may be a spring.

In some embodiments, the apparatus includes a threaded adapter disposable within the pressing member receiving end of the outer sleeve for selectively coupling the second end cap engageable with the pressing member to the outer sleeve. The second end cap may include grooves configured to receive threads of the threaded adapter. The elastic member may extend between a seat portion of the threaded adapter and a seat portion of the pressing member.

In some embodiments of the disclosed apparatus, the inner sleeve of the disclosed apparatus includes a material, within a reservoir in the inner sleeve, which contacts the wounded, damaged, or injured tissue when the wounded, damaged, or injured tissue is inserted in the apparatus. The material of the inner sleeve may comprise a hydrogel that moistens the wounded, damaged, or injured tissue. The material additionally may comprise therapeutic compositions that promote tissue regeneration and/or healing and/or antimicrobial agents.

In yet another aspect, the disclosed apparatus includes an inner sleeve including an end having a first opening sized to receive the wounded, damaged, or injured tissue, an engagement receiving end having a second opening, the inner sleeve defining an internal chamber that extends between the first opening and the second opening, the internal chamber being sized to receive the wounded, damaged, or injured tissue of the subject. The apparatus further includes a matrix disposed in the internal chamber of the inner sleeve, where the matrix comprises a porous scaffold having pores that form substantially aligned channels. The matrix may comprise and/or may be formed from materials including, but not limited to, polymeric material such as proteins, which may include but are not limited to collagen and silk fibroin in any suitable form. The aligned channels of the porous scaffold may be arranged parallel with the longitudinal axis of the inner sleeve. The matrix may include therapeutic compositions as disclosed herein such as regenerative compositions or regenerative cocktails.

In some embodiments, the substantially aligned channels are substantially parallel or parallel to a longitudinal axis in the inner sleeve orthogonal to the first and second openings. In some embodiments, the internal chamber comprises a reservoir of an aqueous solution or dispersing media between the filter media and the protein matrix.

In some embodiments, the apparatus further includes filter media sized to enclose the second opening on the engagement receiving end of the inner sleeve. In some embodiments, the filter medium has a pore size which is large enough to permit air to pass into the apparatus but small enough to prevent microbes from entering the apparatus.

In some embodiments, the internal chamber of the inner sleeve of the provided apparatus includes the provided therapeutic composition.

In another aspect, the present disclosure provides a method of promoting tissue regeneration in a mammal. The method comprises attaching the provided apparatus to a wounded appendage or tissue of a mammal.

In another aspect, the present disclosure provides a use of the apparatus for stimulation of regeneration of tissue in a mammal in need thereof.

The foregoing and other embodiments and advantages of the disclosure will appear from the following description. In the description, reference is made to the accompanying drawings that form a part hereof, and in which there is shown by way of illustration a non-limiting example embodiment. This embodiment does not necessarily represent the full scope of the disclosure, however, and reference is therefore made to the entire disclosure herein for interpreting the scope of the disclosure.

### BRIEF DESCRIPTION OF THE DRAWINGS

The disclosure will hereafter be described with reference to the accompanying drawings, wherein like reference numerals denote like elements.
FIG. 1 is a schematic representation of a side sectional, partially exploded view of an apparatus for stimulation of tissue regeneration at a wound site of a subject according to some embodiments of the present disclosure.
FIG. 2 is a schematic representation of a side sectional, partially exploded view of an apparatus for stimulation of tissue regeneration at a wound site of a subject according to some embodiments of the present disclosure.
FIG. 3 is a diagrammatic representation of a mouse digit showing an amputation line.
FIG. 4 is a schematic representation of a side sectional view of an apparatus for stimulation of tissue regeneration at a wound site of a subject having a moveable pressing member in accordance with some embodiments of the present disclosure.
FIG. 5 is a schematic representation of a side sectional view of the apparatus of FIG. 4 in a compressed state in accordance with some embodiments of the present disclosure.
FIG. 6 a schematic representation of the apparatus of FIG. 1 but including an electrical stimulation device in accordance with some embodiments of the present disclosure.
FIG. 7 is diagrammatic representation of the apparatus of FIG. 6 mounted to a subject in accordance with some embodiments of the present disclosure.
FIG. 8A is a representation of an SEM showing a silk fibroin scaffold with channel-like pores aligned along a long axis in accordance with some embodiments of the present disclosure.
FIG. 8B is a representation of an SEM showing silk scaffolds with pores aligned perpendicular to the long axis in accordance with some embodiments of the present disclosure.
FIG. 8C illustrates a collagen scaffold with pores aligned along the long axis in accordance with some embodiments of the present disclosure.
FIG. 8D illustrates a collagen scaffold with pores aligned perpendicular to the long axis in accordance with some embodiments of the present disclosure.
FIG. 9 is a graphic representation of the length of right hindlimbs as a function of months post amputation (mpa) after exposure to one of the following treatment conditions (i) after an initial 24-hour exposure to a provided multi-drug treatment (MDT) composition, (ii) after exposure to a biodome device for 24-hours, and (iii) no treatment.
FIG. 10 is a graphic representation of touch responses after 17 mpa as assessed by Von Frey Filaments at the distal tip of the regenerate that has been exposed to one of the following treatment conditions (i) after an initial 24-hour exposure to a provided multi-drug treatment (MDT) composition, (ii) after exposure to a biodome device for 24-hours, and (iii) no treatment. Means and SD are presented.
FIG. 11 is a graphic representation of the bone length of right hindlimbs as assessed by microCT and X-ray images as a function of months post amputation (mpa) after exposure to one of the following treatment conditions (i) after an initial 24-hour exposure to a provided multi-drug treatment (MDT) composition, (ii) after exposure to a biodome device for 24-hours, and (iii) no treatment.
FIG. 12 is a graphic representation of bone volume of right hindlimbs as assessed by microCT and X-ray images as a function of months post amputation after exposure to one of the following treatment conditions (i) after an initial 24-hour exposure to a provided multi-drug treatment (MDT) composition, (ii) after exposure to a biodome device for 24-hours, and (iii) no treatment.
FIG. 13 is a graphic representation of number of ATT+ nerve bundles as measured by acetylated α-tubulin (AAT) staining in the right hindlimb following 18 mpa amputation and after exposure to one of the following initial treatment conditions (i) an initial 24-hour exposure to a provided multi-drug treatment (MDT) composition, (ii) after exposure to a biodome device for 24-hours, and (iii) no treatment.
FIG. 14 is a graphic representation of nerve bundle diameter (mm) as measured by acetylated α-tubulin (AAT) staining in the right hindlimb following 18 mpa, and after exposure to one of the following initial treatment conditions (i) an initial 24-hour exposure to a provided multi-drug treatment (MDT) composition, (ii) after exposure to a biodome device for 24-hours, and (iii) no treatment.
FIG. 15 is a graphic representation of particle complexity (pixel²) as assessed by fibronectin expression following 18 mpa and after exposure to one of the following initial treatment conditions (i) an initial 24-hour exposure to a provided multi-drug treatment (MDT) composition, (ii) after exposure to a biodome device for 24-hours, and (iii) no treatment.
FIG. 16 is a graphic representation of the number of Laminin/smooth muscle actin (SMA+) bundles as measured by laminin and SMA+ expression in regenerates obtained 18 mpa, and after exposure to one of the following initial treatment conditions (i) an initial 24-hour exposure to a provided multi-drug treatment (MDT) composition, (ii) after exposure to a biodome device for 24-hours, and (iii) no treatment.
FIG. 17 is a graphic representation of wound closure diameter (cm) obtained 0.5 mpa, and after exposure to one of the following initial treatment conditions (i) an initial 24-hour exposure to a provided multi-drug treatment (MDT) composition, (ii) after exposure to a biodome device for 24-hours, and (iii) no treatment.
FIG. 18 is a graphic representation of the number of SOX2+ cells in the wound site obtained 0.5 mpa, and after exposure to one of the following initial treatment conditions (i) an initial 24-hour exposure to a provided multi-drug treatment (MDT) composition, (ii) after exposure to a biodome device for 24-hours, and (iii) no treatment.

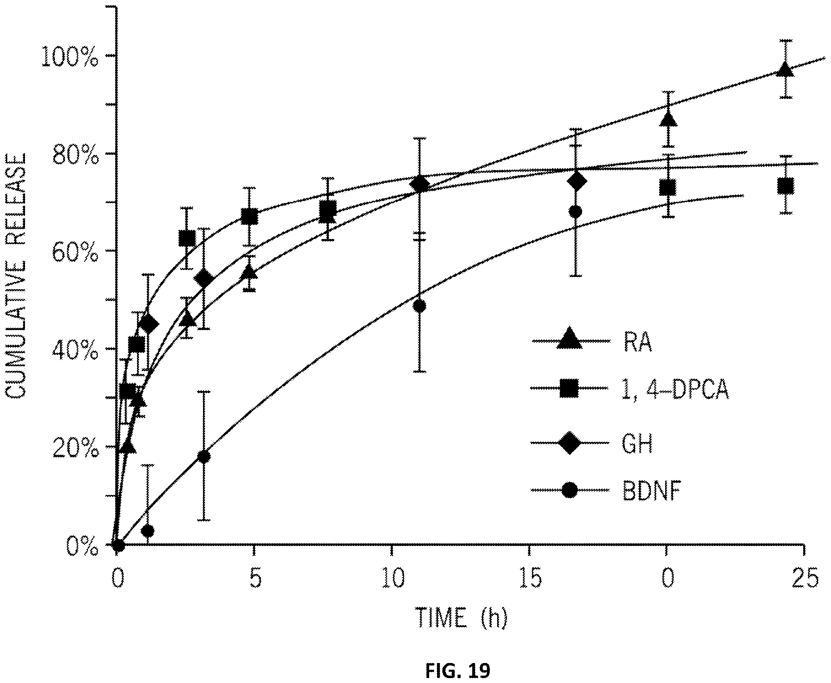

FIG. 19 is a graphic representation of cumulative release for drugs from the provided MDT compositions.

### DETAILED DESCRIPTION

The disclosures of these patents, patent applications, and publications in their entireties are hereby incorporated by reference into this application in order to more fully describe the state of the art as known to those skilled therein as of the date of the invention described and claimed herein. The instant disclosure will govern in the instance that there is any inconsistency between the patents, patent applications, and publications and this disclosure.

Before any embodiments of the disclosure are explained in detail, it is to be understood that the disclosure is not limited in its application to the details of construction and the arrangement of components set forth in the following description or illustrated in the following drawings. The disclosure is capable of other embodiments and of being practiced or of being carried out in various ways.

It is to be understood that the phraseology and terminology used herein is for the purpose of description and should not be regarded as limiting. The use of "including," "comprising," or "having" and variations thereof herein is meant to encompass the items listed thereafter and equivalents thereof as well as additional items. Unless specified or limited otherwise, the terms "mounted," "connected," "supported," and "coupled" and variations thereof are used broadly and encompass both direct and indirect mountings, connections, supports, and couplings. Further, "connected" and "coupled" are not restricted to physical or mechanical connections or couplings.

As used herein, the term "tissue" is defined as an ensemble of similar cells and their extracellular matrix from a same origin that together carry out a specific function. As used herein, "tissue" may be present on an appendage, including, but not limited to phalanges (such as fingers and toes), arms, legs, and the like. As used herein, "tissue" may be present on an organ (such as liver, lung, pancreas, and the like).

The terms "therapeutic composition" "regenerative compositions", "regenerative cocktail" and "multi-drug therapeutic compounds" or "MDT" encompass the forumulates comprising a combination of therapeutic drugs which stimulate or initiate tissue regeneration, and in some embodiments, have a synergistic effect when administered to s subject,

The following discussion is presented to enable a person skilled in the art to make and use embodiments of the disclosure. Various modifications to the illustrated embodiments will be readily apparent to those skilled in the art, and the generic principles herein can be applied to other embodiments and applications without departing from embodiments of the disclosure. Thus, embodiments of the disclosure are not intended to be limited to embodiments shown, but are to be accorded the widest scope consistent with the principles and features disclosed herein. The following detailed description is to be read with reference to the figures, in which like elements in different figures have like reference numerals. The figures, which are not necessarily to scale, depict selected embodiments and are not intended to limit the scope of embodiments of the disclosure. Skilled artisans will recognize the examples provided herein have many useful alternatives and fall within the scope of embodiments of the disclosure.

### Apparatus for Tissue Regeneration

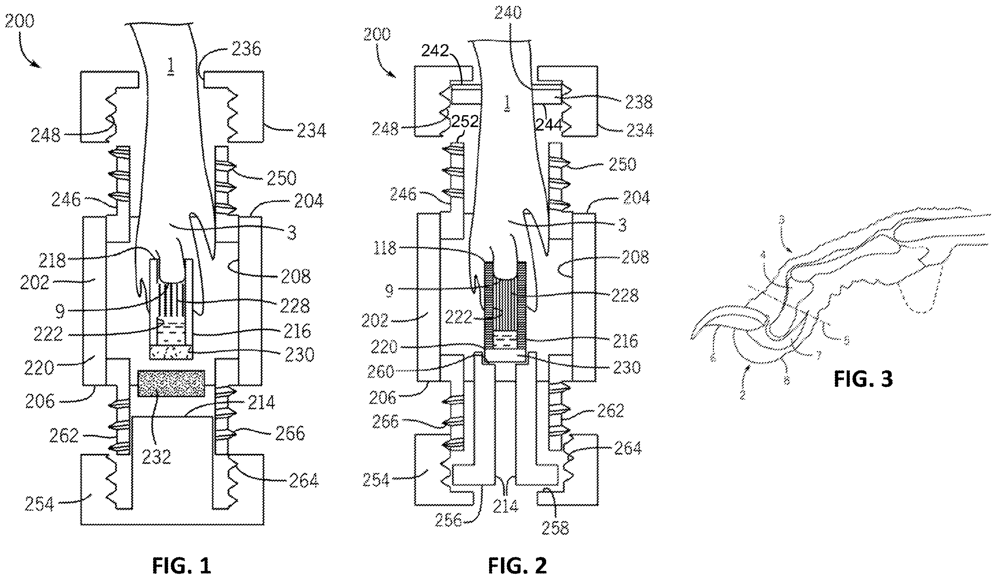

The present disclosure provides apparatus for aiding in tissue regeneration. Referring to FIGS. 1-3, an apparatus 200 for stimulation of tissue regeneration of a subject 1 is shown. In some embodiments, the apparatus 200 is used to enclose a wounded or injured tissue 9 of a subject 1. The wounded or injured tissue 9 may located on an external or internal location of the subject 1. In the illustrated embodiment, the apparatus 200 is described with respect to stimulating tissue regeneration of wounded tissue 9 located on an appendage 3 (*e.g.,* mouse digit) in which the tip 2 has been amputated along a line 5 through at least a portion of the distal phalange 4, whereby regenerated tissue may include among other tissues, bone tissue 6, muscle tissue 7, and skin tissue 8. For exemplary purposes in describing FIGS. 1-7, reference may be made to an "appendage" as comprising exemplary "tissue."

In some embodiments, the wounded or injured tissue 9 for stimulation of tissue regeneration using the apparatus 200 includes epithelial tissue, connective tissue, muscular tissue, or nervous tissue. Exemplary wounded or injured tissue 9 for regeneration includes, but is not limited to, squamous epithelium, cuboidal epithelium, transitional epithelium, pseudostratified columnar epithelium, columnar epithelium, glandular epithelium, bone, tendons, ligaments, adipose, areolar tissue, blood tissue, visceral muscle, smooth muscle, skeletal muscle, cardiac muscle, and neural tissues.

Referring to FIGS. 1-2, the apparatus 100 includes an outer sleeve 202 that extends between an appendage receiving end 204 and a pressing member receiving end 206. In some embodiments, the outer sleeve 202 is a hollow cylinder that defines an internal chamber 208 that is sized to receive the tissue 3 to be treated (e.g., appendage) and the wounded or injured tissue 9. The internal chamber 208 forms a passage that extends between a first opening on the appendage receiving end 204, and a second opening on the pressing member receiving end 206. In some embodiments, the first opening on the appendage receiving end 204 is sized to receive the appendage 3, and the second opening sized to receive a pressing member 214.

The outer sleeve 202 may be formed from a transparent material to permit observation of the wound 9 while the apparatus 200 is in use. In some embodiments, the outer sleeve 202 is sufficiently rigid to prevent any deflection or indentation of the body walls during use to ensure that the desired wound space volume is maintained, and to protect the wounded or injured tissue 9. Exemplary materials of construction for the outer sleeve 202 include, but are not limited to, transparent nylon tubing. The outer sleeve 202 may include one or more openings (not shown) to facilitate replacement of fluid within the internal chamber 208 of the outer sleeve 202. For example, the one or more openings may include a septum that allows a needle to enter and replace fluid within the internal chamber 208.

The apparatus 200 includes an inner sleeve 216 that extends between a wound receiving end 218 and an engagement receiving end 220 opposite the wound receiving end 218. In some embodiments, the inner sleeve 216 is a hollow cylinder that defines an internal chamber 222 sized to receive the wound 9. The internal chamber 222 forms a passage that extends between a first opening on the wound receiving end 218, and a second opening on the engagement receiving end 220. When the apparatus 200 is assembled, the inner sleeve 216 is configured to enclose the wound 9, and the pressing member 214 is configured to bias the engagement receiving end 220 towards the appendage 3 such that the wound 9 is placed in contact with at least a portion of the internal chamber 222. In some embodiments, the pressing member 214 places the wound 9 in contact with a protein matrix 228 disposed within the internal chamber 222 of the inner sleeve 216. The protein matrix 228 may guide tissue growth directionally and/or provide therapeutic agents for the wound 9 to stimulate tissue regeneration.

In some embodiments, the engagement receiving end 220 includes porous filtration media 230 that seals the second opening of the inner sleeve 216. Incorporating porous filtration media 230 in the apparatus 200 prevents contamination and allows for air and media exchange with the surrounding environment. The porous filtration medium 230 helps to keep the wound 9 moist and cell viability high, while reducing necrosis. A compressible member or media exchange member 232 may be positioned between the filter media 230 and the pressing member 214 to provide a reservoir of an aqueous solution or dispersing media that is in fluid communication with the internal chamber 222. In some embodiments, the outer sleeve 202 may comprise an aqueous solution or dispersing medium, which may be placed in fluid communication with the internal chamber 222 of the inner sleeve 216 via the filtration media 230. In some embodiments, the compressible member or media exchange member 232 comprises a gel comprising or cotton optionally wetted with the aqueous solution or dispersing medium. In some embodiments, the protein matrix 228 is displaced from the filtration media 230 by a reservoir of the aqueous solution or dispersing media.

Suitable aqueous solutions or dispersing media include, but are not limited to, water, cell culture medium, buffers (*e.g*., phosphate buffered saline), polyol (for example, glycerol, propylene glycol, liquid polyethylene glycol, and the like), and suitable mixtures thereof. In some embodiments, the dispersing medium includes a therapeutic agent.

The apparatus 200 includes a first end cap 234 engageable with the appendage receiving end 204 of the outer sleeve 202. The first end cap 234 includes an opening 236 that is sized to receive the appendage 3 of the subject 1. In some embodiments, a gasket or septum 238 may be positioned between the first end cap 234 and the appendage receiving end 204 of the outer sleeve 202. The gasket or septum 238 includes a through hole 240 that is sized to receive the appendage 3, and provides a seal that prevents liquid from escaping the internal chamber 208 of the outer sleeve 202. In some embodiments, the septum 238 includes flexible side 242 composed of silicon, and a rigid side 244 comprising polytetrafluoroethylene (PTFE).

The apparatus 200 may include a first adjustable adapter 246 disposable within the appendage receiving end 204 for selectively coupling the first end cap 234 engageable with the appendage receiving end 204 to the outer sleeve 202. In some embodiments the adjustable adapter 246 is a threaded adapter and the first end cap 234 includes grooves 248 to receive threads 250 of the adjustable adapter 246. When assembled, the first end cap 234 may be tightened such that the gasket or septum 238 is placed in contact with a leading end 252 of the adjustable adapter 246 to secure the appendage 3 within the outer sleeve 202.

The apparatus 200 includes a second end cap 254 that is engageable with the pressing member receiving end 206 of the outer sleeve 202. The second end cap 254 is coupled to the pressing member 214 to bias the pressing member 214 toward the engagement receiving end 220 of the inner sleeve 216. Referring to FIG. 1, the pressing member 214 may be directly attached to or form part of the second end cap 254. Referring to FIG. 2, the pressing member 214 may be separate from the second end cap 254. In some embodiments, the pressing member 214 includes an engagement end 256 configured to rest on a seat portion 258 of the second end cap 254. In some embodiments, the pressing member 214 includes a seat portion 260 opposite the engagement end 256 that is configured to receive the engagement end 220 of the inner sleeve 216. The pressing member 214 may be formed of a rigid material or an elastic material that deforms or compresses in response to the growth of the tissue at the wound 9.

The apparatus 200 may include a second adjustable adapter 262 disposable within the pressing member receiving end 206 for selectively coupling the second end cap 254 engageable with the pressing member receiving end 206 to the outer sleeve 202. In some embodiments, the second adjustable adapter 262 is a threaded adapter and the second end cap 254 includes grooves 264 configured to receive threads 266 of the second adjustable adapter 262. The second end cap 254 may be adjusted to control the pressure at the wound 9. One disadvantage of conventional devices is a lack of control of pressure at the wound 9 interface, which leads to variability in tissue regeneration outcomes if there is any type of gap (fluid collection, air, etc.). The apparatus 200 advantageously provides tunable pressure sufficient to hold the protein matrix 228 in contact with the wound 9. In addition, unlike conventional devices, the apparatus 200 facilitates long term attachment (weeks, months, years, or longer) and is adjustable to facilitate growth of the regenerating tissue.

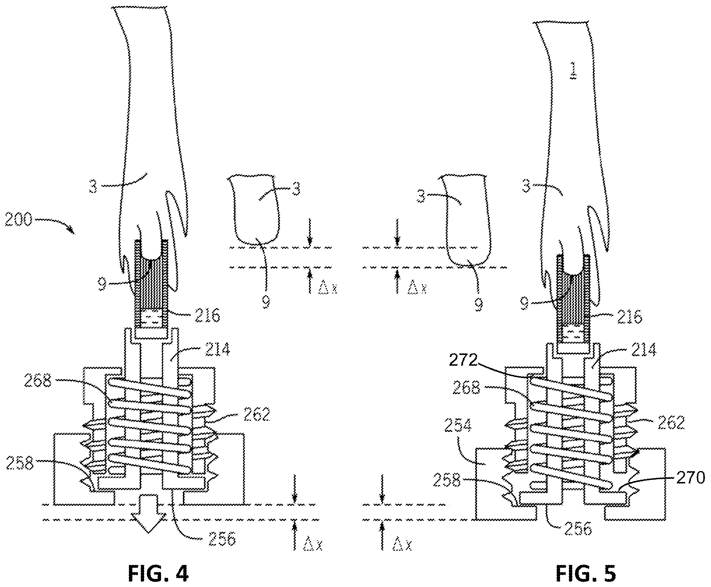

Referring to FIGS. 4-5, in some embodiments, the apparatus 200 includes a moveable or adjustable pressing member 214. For example, the pressing member 214 may be moveable or extendable in response to tissue growth (indicated in FIGS. 4 and 5 by Δx). In some embodiments, an elastic member 268 is configured to extend between a seat portion 270 on the pressing member 214 and a seat portion 272 on the second adjustable adapter 262. In some embodiments, the elastic member 268 (e.g., spring, compressible material, deformable material) generates a resistance force that creates a backward linear translation (e.g., when the second end cap 254 is unscrewed). In some embodiments, the adjustable pressing member 214 includes interlocking spacers that can be extended over time in response to the tissue growth, or a screw-extension system.

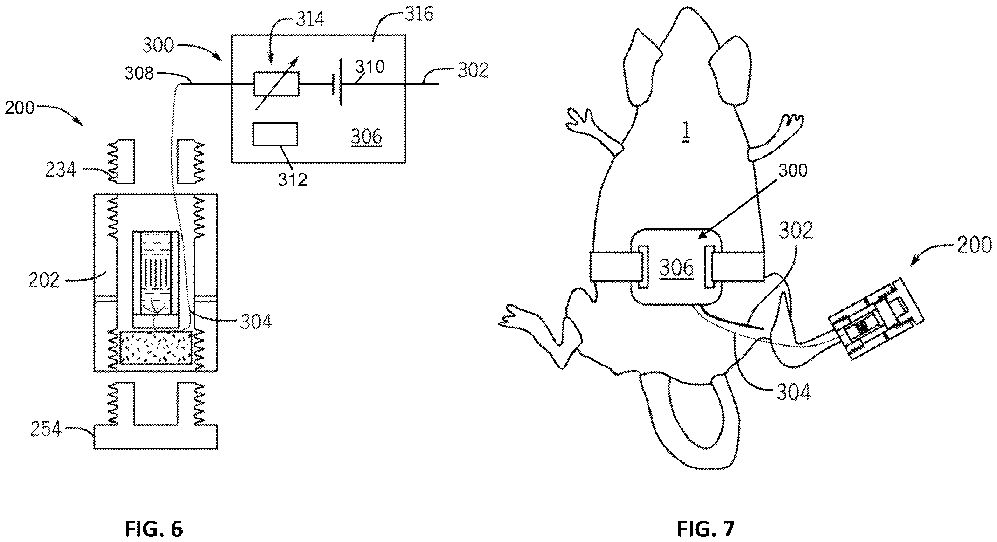

Referring to FIGS. 6-7, in some embodiments, the apparatus 200 also includes an electrical stimulation device 300 to establish a longitudinal electrical field through the wound site 9, which is considered to provide an internal wound stump current and to provide electrical guidance cues for innervation and migration of cell types near the wound site. The electrical stimulation device 300 includes an anode 302 and a cathode 304 that are electrically connected to corresponding terminals of a power source 306 through leads 308, 310.

In some embodiments, cathode 304 is in the form of a stainless steel wire which is disposed adjacent the wound 9. A portion of the cathode 304 resides outside the apparatus 200 and is connectable to the lead 308, and a portion of the cathode 304 resides within the internal chamber 222 of the inner sleeve. The anode 302 is a conductive wire that may be inserted in the subject 1 at a location distant from the wound site 9. In the illustrated embodiment in which the apparatus 200 is disposed on an appendage 3, the anode 302 is disposed in the upper portion of the limb (rear leg) from which the appendage 3 extends. The anode 302 may comprise a Platinum/Iridium alloy wire which is connected to the power source 306 via the lead 310. The anode 302 can be permanently implanted, or temporarily inserted as needed.

The power source 306 includes a battery pack 312 and circuitry 314, both of which are enclosed in a housing 316 and configured to provide a constant, low level current to the electrodes 302, 304 when connected thereto. In the embodiment illustrated in FIG. 6, the power source 306 resides externally of the subject 1, and the electrodes 302, 304 are configured to be detachably connectable to the power source 306. In this arrangement, when electrical stimulation is used, the cathode 304 and anode 302 are electrically connected to the power source 306 for the duration of the electrical stimulation treatment, and then disconnected between electrical stimulation treatments. Since the power source 306 and leads 308, 310 may be detached from the respective electrodes 302, 304, this arrangement conveniently reduces the overall bulk of the combined apparatus 200 and electrical stimulation device 300 during treatment paradigms in which electrical stimulation is used only intermittently.

In some embodiments, the protein matrix 228 comprises a biocompatible polymer. The biocompatible polymer suitable for use with the apparatus 100 includes, but is not limited to, polyethylene oxide (PEO), polyethylene glycols (PEGs), collagen, fibronectin, keratin, polyaspartic acid, polylysine, alginate, chitosan, chitin, hyaluronic acid, pectin, polycaprolactone, polylactic acid, polyglycolic acid, polyhydroxyalkanoates, dextrans, polyanhydrides, polymer, PLA-PGA, polyanhydride, polyorthoester, polycaprolactone, polyfumarate, collagen, silk fibroin, chitosan, alginate, hyaluronic acid and other biocompatible and/or biodegradable polymers. In some embodiments, the protein matrix 228 is silk fibroin and/or collagen.

In some embodiments, the protein matrix 228 is processed from silk solutions (e.g., aqueous solutions) that have a silk solution concentration between about 1% silk to about 50% silk. In some embodiments, the silk fibroin-based materials are processed from silk solutions to form varied material formats, such as fibers, foams, particles, films, and/or hydrogels.

In some embodiments, the protein matrix 228 is porous or has a porosity. As used herein, the term "porosity" may refer to a measure of void spaces in a material and is a fraction of volume or voids over the total volume, as a percentage between 0 and 100%. A determination of a porosity is known to a skilled artisan using standard techniques, for example, mercury porosimetry and gas adsorption (*e*.*g*., nitrogen adsorption).

In some embodiments, the protein matrix 228 includes pores that match tissue surface areas (size) of the tissue upon which it is affixed in the apparatus 200 to optimize regrowth of the wounded or injured tissue. In some embodiments, the protein matrix 228 has a pore size between about 1 µm to about 1500 µm, or about 350 µm, about 400 µm, about 450 µm, about 500 µm, about 550 µm, about 600 µm, about 650 µm, about 700 µm, about 750 µm, about 800 µm, about 850 µm, about 900 µm, about 950 µm, about 1000 µm, about 1050 µm, about 1100 µm, about 1150 µm, about 1200 µm, about 1300 µm, about 13350 µm, about 1400 µm, about 1450 µm, or about 1500. In terms of pore size, generally, about 100 µm to about 300 µm or about 100 µm, about 150 µm, about 200 µm, about 250 µm, or about 300 µm is suitable to support sufficient oxygen, nutrient, and waste transport, while providing a suitable niche for cell and tissue growth. In other embodiments, smaller pore diameters, such as about 50 µm to about 100 µm, or about 50 µm, about 60 µm, about 70 µm, about 80 µm, about 90 µm, or about 100 µm is suitable smaller tissue such as nerves or blood cells. Higher porosity may facilitate improved tissue outcomes due to improved nutrient transfer and waste removal.

The protein matrix 228 may have pores that form a directional pattern that is used to guide the growth the tissue at the wound 9. In some embodiments, the directional pattern includes aligned pores that formed substantially aligned channels. The aligned pores may be arranged in to be parallel with a longitudinal axis of the inner sleeve 216. In some embodiments, the aligned pores are formed by freezing a protein solution (*e.g*., silk or collagen solution) on a conductive substrate (*e.g*., aluminum plate) with a steep temperature gradient induced by merging the conductive substrate with a cold source (*e.g*., liquid nitrogen). It is contemplated that finger-like columns of ice crystals growing from the cold surface create a channel-like structure internally inside the frozen protein. The frozen protein is then lyophilized over a duration (*e.g*., 24 hours) to remove the water. The resultant product is a protein matrix 228 having substantially aligned pores.

In some aspects, the protein matrix comprises silk fibroin. As used herein, "silk fibroin" or "SF", may refer to a biopolymer produced from silkworm fibroin and insect or spider silk protein. For example, silk fibroin useful for the present disclosure may be that produced by a number of species, including, without limitation: *Antheraea mylitta; Antheraea pernyi; Antheraea yamamai; Galleria mellonella; Bombyx mori; Bombyx mandarina; Galleria mellonella; Nephila clavipes; Nephila senegalensis; Gasteracantha mammosa; Argiope aurantia; Araneus diadematus; Latrodectus geometricus; Araneus bicentenarius; Tetragnatha versicolor; Araneus ventricosus; Dolomedes tenebrosus; Euagrus chisoseus; Plectreurys tristis; Argiope trifasciata; and Nephila madagascariensis.* Alternatively, silk utilized in the present disclosure may be prepared through an artificial process, for example, involving genetic engineering of cells or organisms (*e.g*., genetically engineered bacteria, yeast, mammalian cells, non-human organisms, including animals, or transgenic plants).

SF is a structural protein, like collagen, but with a unique feature: it is produced from the extrusion of an amino-acidic solution by a living complex organism into the external environment, while collagen is produced *in vivo,* in the extracellular space by self-assembly of cell-produced monomers and not secreted to the external environment. SF properties are derived from its structure, which consists of hydrophobic blocks staggered by hydrophilic, acidic spacers. In its natural state, SF is organized into semicrystalline materials with β-sheet crystals alternated with amorphous regions, which provide strength and resilience to the protein materials formed from the protein. The multiplicities of forms in which regenerated SF can be processed at a low to high protein concentration and low to high molecular weight make it attractive for several high-tech applications.

Processing of SF generally involves the partial or total dehydration of a fibroin solution (protein content of about 1wt % to about 15 wt %) to form, *e.g.,* films, sponges, gels, spheres (micron- to nano-sized) and foams with numerous techniques (e.g. solvent casting, freeze drying, salt leaching, sonication). These fabrication processes provide a robust material that combines mechanical strength with biochemical properties.

The silk fibroin solutions used in methods and compositions provided herein may be obtained from a solution containing a dissolved silkworm silk, such as, for example, from *Bombyx mori.* Alternatively, the silk fibroin solution may be obtained from a solution containing a dissolved spider silk, such as, for example, from *Nephila clavipes.* The silk fibroin solution can also be obtained from a solution containing a genetically engineered silk such as from bacteria, yeast, mammalian cells, transgenic animals or transgenic plants. See, for example, WO 97/08315 and US Patent 5,245,012. Genetically engineered silk can, for example, also comprise a therapeutic agent, *e.g.,* a fusion protein with a cytokine, an enzyme, or any number of hormones or peptide- based drugs, antimicrobials and related substrates.

Silk fibroin solution can be prepared by any conventional method known to one skilled in the art. In some embodiments, a silk solution is an aqueous silk solution. In other embodiments, silk solutions may contain a second polymer to facilitate transitions to the solid state (e.g., polyethylene glycol, collagen, hyaluronic acid, and the like.).

Silkworm cocoon silk contains two structural proteins, the fibroin heavy chain (~ 350 kDa); and the fibroin light chain (about 25 kDa), which are associated with a family of non-structural proteins termed sericins, that glue the fibroin chains together in forming the cocoon. The heavy and light fibroin chains are linked by a disulfide bond at the C-terminus of the two subunits (see Takei, et al., J. Cell Biol., 105: 175, 1987; see also Tanaka, et al,. J. Biochem. 114: 1, 1993; Tanaka, et al., Biochim. Biophys. Acta., 1432: 92, 1999; Kikuchi, et al., Gene, 110: 151, 1992). The sericins are a high molecular weight, soluble glycoprotein constituent of silk which gives the stickiness to the material. These glycoproteins are hydrophilic and can be easily removed from cocoons by boiling in water "degumming").

In some embodiments, silk polypeptide compositions utilized in accordance with the present compositions are substantially free of sericins (*e.g*., contain no detectable sericin or contain sericin at a level that one of ordinary skill in the pertinent art will consider negligible for a particular use).

In one exemplary method of obtaining silk polypeptide compositions, *B. mori* cocoons are boiled for about 30 minutes in an aqueous solution, such as, but not limited to, about 0.02M Na₂CO₃. The boiling (degumming) time is in a range of about 5 minutes to about 120 minutes and the boiling (degumming) temperature is in a range of about 30°C to about 120°C. The cocoons may be rinsed, for example, with water to extract the sericin proteins and the extracted silk is dissolved in an aqueous salt solution. Exemplary non-limiting salts useful for this purpose include lithium bromide, lithium thiocyanate, calcium nitrate, and other chemicals capable of solubilizing silk. For example, the extracted silk is dissolved in about 9M to about 12 M LiBr solution. The salt is then removed, for example, by dialysis.

If desired, the solution can then be concentrated using, any method known in the art. For example, dialysis against a hygroscopic polymer, for example, PEG, a polyethylene oxide, amylose or sericin can be done. PEG having a molecular weight of about 8,000 g/mol to about 10,000 g/mol and has a concentration of about 25% to about 50%. Any dialysis system can be used, *e.g.,* a slide-a-lyzer dialysis cassette (Pierce, MW CO 3500). The solution is dialyzed for a time period sufficient to result in a final concentration of aqueous silk solution of between about 1% to about 30%. In some cases, dialysis for about 2 hours to about 12 hours is sufficient.

In some embodiments, the present disclosure provides a method of attaching the apparatus 200 to an appendage or tissue of a subject in need of tissue regeneration. The method includes contacting a wounded appendage or tissue 9 of the subject 1 to the wound receiving end 218 of the inner sleeve 216. The wounded appendage or tissue 9 may be placed in contact, or adjacent to, the protein matrix 228 comprising the provided therapeutic compositions. In some embodiments, prior to contacting the wounded appendage or tissue 9 to the protein matrix 228, the wounded appendage or tissue 9 is slid through the gasket or septum 238 and the opening 236 of the first end cap 234.

The method further includes placing the appendage 3 of the subject through the appendage receiving end 204 of the outer sleeve 202 so that the inner sleeve 216 is positioned within the internal chamber 208 of the outer sleeve 202. In some embodiments, the method includes selectively engaging the first end cap 234 and the second end cap 254 to the outer sleeve 202 such that the pressing member 214 biases the engagement receiving end 220 towards the appendage 3. In some embodiments, the method includes biasing the pressing member 214 towards the appendage 3 such that the wound 9 is placed in contact with at least a portion of the internal chamber 222 of the inner sleeve. The contact pressure between the wounded appendage or tissue 9 and the protein matrix 228 may be adjusted by selectively engaging or disengaging the second end cap 254 (*e.g.*, tightening or loosening the second end cap 254 via grooves 264 and threads 266).

In some embodiments, the wounded appendage or tissue 9 is maintained within the apparatus 200 for a duration to promote tissue regeneration. In some embodiments, the duration is about 1 minute, or about 10 minutes, or about 30 minutes, or about 1 hour, or about 2 hours, or about 3 hours, or about 4 hours, or about 5 hours, or about 6 hours, or about 12 hours, or about 24 hours, or 2 days, or about 3 days, or about 4 days, or about 5 days, or about a week, or about two weeks, or about three weeks, or about a month, or about six months, or about one year, or within a duration range bounded by any of these values. During the course of the duration, the protein matrix 228 may be kept moist by adding or replacing a buffered solution within the inner sleeve 216.

### Therapeutic Compositions for Tissue Regeneration

Also disclosed herein are therapeutic compositions for tissue regeneration and "multi-drug treatment" compositions (MDT). The therapeutic compositions may be utilized alone or in conjunction with the disclosed apparatus.

The therapeutic compositions used with the apparatus according to the invention may be any composition that stimulates, initiates, or directly or indirectly aids in tissue regeneration. Alternatively, the therapeutic compositions according to the invention may be a combination of components which act synergistically to stimulate or initiate or directly or indirectly aid in tissue regeneration. For example, the provided components (e.g., two or more of the components, such as a growth factor, an inhibitor of prolyl hydroxylase domain (PHD) enzyme, vitamin A or a derivative thereof, lipid mediator, or peptide/protein hormone) in the therapeutic compositions act synergistically to increase the rate of regeneration (e.g., increase tissue regeneration as measured by soft tissue length, bone length, bone volume, increased touch response, number of ATT+ nerve bundles, diameter of ATT+ nerve bundles, regenerate particle complexity by fibronectin expression, number of laminin/SMA+ bundles, reduced wound diameter at start of treatment, number of SOX2+ cells) at the wounded appendage or tissue site relative to control experiments with no treatment.

In some embodiments, at least two of the components in the provided therapeutic compositions act synergistically to increase the rate of regeneration, or at least three of the components, or at least four of the components, or at least five of the components, or all of the components act synergistically to increase the rate of regeneration.

In one example, the disclosed apparatus may comprise the therapeutic compositions within the inner sleeve (e.g., within a reservoir in the inner sleeve). The therapeutic compositions may be present in a material or matrix that contacts the wound site and the therapeutic compositions may be delivered to the wound site when the apparatus is worn on a subject's appendage. Wearable apparatus that comprise and deliver therapeutic compositions are known in the art. (*See, e.g.,* Herrera-Rincon et al., Cell Reports 25, 1593-1609 (2018).

The disclosed therapeutic compositions may be present in a polymeric material such as, but not limited to, a silk hydrogel material. Methods for loading therapeutic compositions and drugs into a hydrogel material are known in the art. For example, a silk hydrogel material loaded with a therapeutic composition may be prepared as follows. A therapeutic composition may be added to a silk solution (*e.g.,* a 3% w/v silk solution), which then is induced to gel via addition of a reagent such as horseradish peroxidase (*e.g*., to a concentration of about 20 U/ml silk solution) with hydrogen peroxide (*e.g.,* to a concentration of 0.01% w/v). The silk can also gel with this enzymatic reaction, via a drop in pH, addition of energy such as via sonication or vortexing, an applied electric field, or addition of methanol, as some of many options.

The disclosed therapeutic compositions may comprise one more agents that increase axonal/neurite growth and/or general cell proliferation. Preferably, the disclosed therapeutic compositions do not promote pluripotency in cells and/or lead to teratoma formation.

The disclosed therapeutic compositions may comprise one or more agents that promote tissue regeneration and/or healing. In some embodiments, the therapeutic compositions comprise one or more of a growth factor, an agent that inhibits the inhibitor of the hypoxia-inducible factor 1-alpha (HIF1-alpha), vitamin A or a derivative thereof, a lipid mediator such as a metabolic product of omega-3 fatty acids and may be derived from eicosapentaenoic acid or docosahexaenoic acid, a growth hormone, a steroid, and a depolarizing agent.

In some embodiments, the disclosed therapeutic compositions may include growth factors, such as neurotrophic factors. A neurotrophic factor is a protein that promotes the growth and survival of nerve cells during development, and that promotes maintenance of adult nerve cells. (*See, e.g.,* Terenghi , J. Anat. 1999 ;194 ( Pt 1):1-14. Exemplary neurotrophic factors include, but are not limited to, brain-derived neurotrophic factor (BDNF), nerve growth factor (NGF), neurotrophin-3 (NT-3), neurotrophin-4 (NT-4), glial-derived neurotrophic factor (GDNF), ciliary neurotrophic factor (CNTF), leukemia inhibitor factor (LIF), and combinations thereof. The growth factor may be present at a dose within the therapeutic composition of at least about 0.1 µg/ml, about 0.2 µg/ml, about 0.3 µg/ml, about 0.4 µg/ml, about 0.5 µg/ml, about 0.6 µg/ml, about 0.7 µg/ml, about 0.8 µg/ml, about 0.9 µg/ml, or about 1.0 µg/ml or within a dose range bounded by any of these values. When the growth factor is present in a component of the disclosed apparatus (e.g., when the growth factor is loaded in the inner sleeve or in a component of the inner sleeve), the apparatus may comprise a concentration of the growth factor of at least about 0.1 µg/apparatus, about 0.2 µg/apparatus, about 0.3 µg/apparatus, about 0.4 µg/apparatus, about 0.5 µg/apparatus, about 0.6 µg/apparatus, about 0.7 µg/apparatus, about 0.8 µg/apparatus, about 0.9 µg/apparatus, or about 1.00µg/apparatus or within a concentration range bounded by any of these values. The growth factor promotes the growth of one or more tissue types.

The disclosed therapeutic compositions may include a prolyl hydroxylase domain (PHD) enzyme inhibitor (*i.e.,* a PHD inhibitor), for example, in order to stabilize constitutive expression of the HIF-1α protein. (*See, e.g.,* Ariazi et al., J. Pharmacol. Expt. Therap. (2017), 363 (3) 336-347; and Nangaku et al., Arterioscler., Thromb. Vas. Biol. 2007;27:2548-2554). Suitable PHD inhibitors may include, but are not limited to, 4,4α-dihydro-4-oxo-1,10-phenanthroline-3-carboxylic acid (1,4-DPCA), N-[(1,3-dicyclohexylhexahydro-2,4,6-trioxo-5-pyrimidinyl)carbonyl]-glycine (*i.e*., , GSK1278863 or Daprodustat), 6-Amino-1,3-dimethyl-5-[(2-pyridinylthio)acetyl]-2,4(1H,3H)-pyrimidinedione (*i.e*., , TM6089), 6-Amino-1,3-dimethyl-5-[[2-(2-pyridinyl)-4-quinolinyl]carbonyl]-2,4(1H,3H)-pyrimidinedione (*i.e*., , TM60008), N-[(4-hydroxy-1-methyl-7-phenoxy-3-isoquinolinyl)carbonyl]-glycine (*i.e.,* , FG4592 or Roxadustat), iron chelators, and combinations thereof. Optionally, the PHD inhibitor may be present at a dose within the therapeutic composition of at least about 0.004 µg/ml, about 0.006 µg/ml, about 0.008 µg/ml, about 0.010 µg/ml, about 0.012 µg/ml, about 0.014 µg/ml, about 0.016 µg/ml, 0.018 µg/ml, about 0.020 µg/ml, about 0.022 µg/ml, or 0.024 µg/ml or within a dose range bounded by any of these values. When the PHD inhibitor is present in a component of the disclosed apparatus (*e.g*., when the PHD inhibitor is loaded in the inner sleeve or in a component of the inner sleeve), the apparatus may comprise a concentration of the PHD inhibitor of at least about 0.087 µg/apparatus, about 0.092 µg/apparatus, about 0.097 µg/apparatus, about 0.102, about 0.107 µg/apparatus, about 0.112 µg/apparatus, about 0.117 µg/apparatus, about 0.122 µg/apparatus, about 0.127 µg/apparatus, or about 0.132 µg/apparatus or within a concentration range bounded by any of these values. The PHD inhibitor controls excess collagen deposition at a wound site.

The disclosed compositions may include vitamin A or a metabolite or derivative thereof or any agent that functions in proximo-distal positional information. Exemplary derivatives of vitamin A include, but are not limited, to retinoic acid, retinol, retinyl carboxylates (*e.g.,* retinyl acetate, retinyl propionate, and retinyl palmitate), tretinoin, and tazarotene, and combinations thereof. Agents that may function in proximo-distal position information include, but are not limited to, bone morphogenetic protein 9 (BMP9), nodal growth differentiation factor (*i.e.,,* HTX5 or NODAL), Activin, transforming growth factor-beta (TGF-β), and fibroblast growth factor 8 (FGF8). The vitamin A or a metabolite or derivative thereof or any agent that functions in proximo-distal positional information may be present at a dose within the therapeutic composition of at least about 0.03 µg/ml, about 0.06 µg/ml, about 0.09 µg/ml, about 0.12 µg/ml, about 0.15 µg/ml, about 0.18 µg/ml, about 0.21 µg/ml, about 0.24 µg/ml, or about 0.27 µg/ml or within a dose range bounded by any of these values. When the vitamin A or the derivative thereof (or the agent that functions in proximo-distal positional information) is present in a component of the disclosed apparatus (e.g., when the vitamin A or the derivative thereof or the agent that functions in proximo-distal positional information is loaded in the inner sleeve or in a component of the inner sleeve), the apparatus may comprise a concentration of the vitamin A or the derivative thereof or the agent that functions in proximo-distal positional information of at least about 0.03 µg/apparatus, about 0.06 µg/apparatus, about 0.09 µg/apparatus, about 0.12 µg/apparatus, about 0.15 µg/apparatus, about 0.18 µg/apparatus, about 0.21 µg/apparatus, about 0.24 µg/apparatus, or about 0.27 µg/apparatus or within a concentration range bounded by any of these values.

The disclosed therapeutic compositions may include a lipid mediator and/or a metabolic byproduct of an omega-3 fatty acid that promotes the resolution of the inflammatory response (*i.e.,* an anti-inflammatory agent). Suitable lipid mediators may include derivatives (*e.g*., metabolic byproducts) of omega-3 fatty, and/or derivatives of eicosapentaeonic acid or docosahexaenoic acid that promote the resolution of the inflammatory response (*i.e.*, anti-inflammatory). Exemplary lipid mediators may include but are not limited to resolvins such as resolvin 5, interleukin 6 (IL-6), interleukin 4 (IL-4), tumor necrosis factor-alpha (TNF-alpha), nuclear factor kappa-light-chain-enhancer of activated B cells (NF-kB), and combinations thereof. Optionally, the lipid mediator may be present at a dose within the therapeutic composition of at least about 0.006 µg/ml, about 0.012 µg/ml, about 0.018 µg/ml, about 0.024 µg/ml, about 0.030 µg/ml, about 0.036 µg/ml, about 0.042 µg/ml, about 0.048 µg/ml, or about 0.054 µg/ml or within a dose range bounded by any of these values. When the lipid mediator is present in a component of the disclosed apparatus (*e.g*., when the lipid mediator is loaded in the inner sleeve or in a component of the inner sleeve), the apparatus may comprise a concentration of the lipid mediator of at least about 0.005, µg/apparatus, about 0.011 µg/apparatus, about 0.017 µg/apparatus, about 0.023 µg/apparatus, about 0.029 µg/apparatus, about 0.035 µg/apparatus, about 0.041 µg/apparatus, about 0.047 µg/apparatus, or about 0.053 µg/apparatus or within a concentration range bounded by any of these values.

The disclosed therapeutic compositions may include peptide hormones, for example, peptide hormones which stimulates growth, cell reproduction, and cell regeneration. (*See, e.g.,* Schmidmaier et al., Bone (2002)31(1):165-72; and Schneider et al., J. Clin. Invest. 115 (8): 2083-98. Exemplary hormone peptides or proteins include, but are not limited to, growth hormone (GH), insulin-like growth factor-1 (IGF-1), transforming growth factor-beta-1 (TGFβ-1), epidermal growth factor (EGF), Granulocyte-colony stimulating factor (G-CSF), and fibroblast growth factor FGF. The growth hormone or steroid may be present at a dose within the therapeutic composition of at least about 0.1 µg/ml, about 0.2 µg/ml, about 0.3 µg/ml, about 0.4 µg/ml, about 0.5 µg/ml, about 0.6 µg/ml, about 0.7 µg/ml, about 0.8 µg/ml, about 0.9 µg/ml, or about 1.0 µg/ml or within a dose range bounded by any of these values. When the growth hormone or steroid is present in a component of the disclosed apparatus (e.g., when the growth hormone or steroid is loaded in the inner sleeve or in a component of the inner sleeve), the apparatus may comprise a concentration of the growth hormone or steroid of at least about 0.1 µg/apparatus, about 0.2 µg/apparatus, about 0.3 µg/apparatus, about 0.4 µg/apparatus, about 0.5 µg/apparatus, about 0.6 µg/apparatus, about 0.7 µg/apparatus, about 0.8 µg/apparatus, about 0.9 µg/apparatus, or about 1.0 µg/apparatus or within a concentration range bounded by any of these values.

The disclosed therapeutic compositions may include depolarizing agents. Suitable depolarizing agents may include, but are not limited to ionophores (e.g., an ion channel opener or blocker). Suitable depolarizing agents may include, but are not limited to, monensin, potassium gluconate, sodium gluconate, and the like.

The disclosed therapeutic compositions may be used for treating a subject in need of treatment. As used herein, a "subject" means a human or animal. Usually the animal is a vertebrate such as a primate, rodent, domestic animal or game animal. Primates include chimpanzees, cynomologous monkeys, spider monkeys, and macaques, e.g., Rhesus. Rodents include mice, rats, woodchucks, ferrets, rabbits and hamsters. Domestic and game animals include cows, horses, pigs, deer, bison, buffalo, feline species, e.g., domestic cat, canine species, e.g., dog, fox, wolf, avian species, e.g., chicken, emu, ostrich, and fish, e.g., trout, catfish and salmon. In certain embodiments of the aspects described herein, the subject is a mammal, e.g., a primate, e.g., a human. A subject can be male or female. Preferably, the subject is a mammal. The mammal can be a human, non-human primate, mouse, rat, dog, cat, horse, or cow, but are not limited to these examples. Mammals other than humans can be used as subjects that represent animal models of tissue repair, regeneration and/or reconstruction. In addition, the methods and compositions described herein can be used to treat domesticated animals and/or pets.

In some embodiments, the disclosed therapeutic compositions may be used for treating a wounded or injured appendage or tissue of a subject in need of stimulation of tissue regeneration. The tissue or appendage may be internal or external to the subject. Exemplary wounded or injured tissue within the subject in need for regeneration includes, but is not limited to, squamous epithelium, cuboidal epithelium, transitional epithelium, pseudostratified columnar epithelium, columnar epithelium, glandular epithelium, bone, tendons, ligaments, adipose, areolar tissue, blood tissue, visceral muscle, smooth muscle, skeletal muscle, cardiac muscle, and neural tissues.

In some embodiments, the disclose provides a method of administering to the subject the disclosed therapeutic compounds comprising the disclosed compounds in an effective amount to regenerate at least a portion of the wounded or injured appendage or tissue. In some embodiments, the method includes contacting the wounded or injured appendage or tissue to the therapeutic compounds, which may or may not be present in a provided hydrogel. In some embodiments, the provided therapeutic compounds or provided hydrogels are contacted to the wounded or injured appendage or tissue within the provided apparatus herein.

The disclosed apparatus and/or therapeutic compositions promote tissue regeneration. Tissue regeneration may be measured by any method known in the art such as, but not limited to, measuring the expression of Yamanaka factors, Sox2, Oct3/4, Klf4, and/or c-Myc in tissue treated with the apparatus according to the disclosure and/or therapeutic compositions versus tissue not treated with the disclosed apparatus and/or therapeutic compositions.

### Animal Model Testing

Anurans that have matured towards adolescent stages can regenerate their amputated or injured limbs when exposed to regeneration inducers delivered through slow release beads implanted in the amputated tissues. However, fully non-regenerative, strongly post-metamorphic (adult) *Xenopus* fail to regenerate their hindlimbs upon amputation, instead generating featureless cartilaginous spikes (Suzuki et al., (2006) TheScientificWorldJOURNAL, 6.). This model was used to test if regeneration inducers could stimulate regeneration.

As described in the EXAMPLES below, a complex intervention on adult *Xenopus* hind-leg amputations were tested to address several aspects of limb regeneration. A wearable bioreactor ("BioDome") was used to attain control over the local microenvironment of a wound *in vivo.* A mechanism was sought whereby a brief exposure period to a regenerative cocktail kickstarts a lengthy endogenous morphogenetic cascade without continuous micromanagement. A variety of stimuli were chosen, which induce pro-regenerative activity, such as agents which reduce inflammation, promote neural sparing, and induce overall growth.

A brief exposure period (*e.g.,* 24 hours) to a wearable bioreactor containing silk infused with several small molecule compounds was found to induce dramatic outgrowth, patterning, and sensorimotor function following amputation in *Xenopus.* Treated animals displayed a marked delay of wound closure, followed by long-term (about 16-month) growth outcomes, including increased bone length, soft tissue patterning, and neuromuscular repair. Histologically, the new limbs contained nerve, smooth muscle indicative of blood vessels, and reorganization of the extracellular matrix proteins involved in remodeling of the limb. Transcriptomic analysis identified immediate and short-term pathways and transcriptional targets of the intervention in the blastema. The RNA-seq test also revealed rapid responses to full treatment devices (as compared to sham controls) in the brain. Regenerated bone displayed anatomical features characteristic of wildtype morphology, and distal limb soft tissue displayed digit-like projections. Moreover, the animals used the newly formed limb to ambulate similar to wild-type frogs. In addition, the sensorimotor pathways were restored in animals exposed to the full treatment condition, indicating tissue-repatterning included the reextension or regrowth of sensory afferents as well as neuromuscular tissue interfaces.

These data demonstrate that adult *Xenopus* are capable of being induced toward very significant, lengthy regenerative response by a brief trigger not requiring gene therapy or stem cell implants, and reveal molecular, cell, and tissue-level components of this process that occur at the wound and at the distant brain.

The following examples set forth, in detail, ways in which the present disclosure may be used or implemented, and will enable one of ordinary skill in the art to more readily understand the principles thereof. The following examples are presented by way of illustration and are not meant to be limiting in any way.

### EXAMPLES

### Animals

Adult female *Xenopus laevis* (n = 115) measuring 5 cm to 6.25 cm (nose-to-tail) (Nasco, Fort Atkinson, WI) were allowed to acclimate to holding tanks for 2 weeks before experimentation. Animals were maintained at 18°C in 10 L plastic tanks containing a defined frog water (Reef Salt, Seachem Laboratories, about1.65 kΩ conductivity, 7.8-8.0 pH) and exposed to 12 hr light-dark cycles. Prior to experimentation, animals were soaked in a broad-spectrum gentamicin antibiotic for 2 hr (Gibco, Fisher Scientific, USA) to minimize bacterial contamination of the limb stump after amputation.

### Limb Amputation

Hindlimb amputation surgeries were carried out according to previously established protocols published by Golding et al. (2016), PLoS One 11, e0155618, and Herrera et al. (2018), Cell Rep 25, 1593-+. Briefly, animals were first anesthetized by full-body immersion in buffered frog water containing 0.05% benzocaine. Upon loss of toe pinch reflex, 75 mg/kg buprenorphine was injected subcutaneously just below the lateral line on the contralateral side to the leg that would be amputated. Right hindlimbs were amputated at the midpoint of the tibiofibular bone with a sterile microsurgical blade using a straight cut. No bone resection was performed, nor was a tissue flap sewn over the wound site. Following hemostasis, animals regained consciousness and were allowed to recover in sterile frog water for a minimum of 60 min.

### Device Attachment

Animals were randomly assigned to one of three treatment conditions: No device, BioDome only, or BioDome with cocktail treatment (described below in "Biodome Fabrication and Cocktail Composition"). The device attachment procedure was preceded by a second anesthetization (0.05% benzocaine soak, 75 mg/kg buprenorphine). Unconscious animals were then fitted with devices that were affixed to the amputation site stump using monofilament surgical sutures (7-0 Monosof, 18" P-16 cutting, Covidien, USA). Two sutures were placed through the dermal layer on either side of the leg. These stitches were sufficient to hold the device in place and did not damage the underlying deep fascial layers. Following attachment, animals were returned to their home tanks whereupon they regained consciousness and were allowed to swim freely. Control animals were handled similarly to those that received devices, but no devices were attached to the wound stump. The results presented herein were produced using a sutured biodome.

Alternatively, an adjustable biodome may be used. An attachment procedure for an adjustable biodome includes pushing the amputated limb at the proper position through the cap and donut-shaped septum, and securing the limb using a bioadhesive Skin-Tite Bioadhesive, Smooth-On, PA). The scaffold-containing biodome insert was attached to the amputated digit and secured with a small amount of the adhesive. The acrylic protective cap was screwed into the top cap followed by pushing the insert closer to the wound bed with the custom washer and closed with the second cap. To keep the tissue moist over the attachment period, about4 µL of sterile phosphate buffer saline (PBS) 1X was slowly injected into the insert using a 31G needle. PBS was changed every 2 d to remove the cell waste and keep the tissue fresh. Devices were kept attached until animals were sacrificed for subsequent analyses.

### BioDome Fabrication

The sutured biodome was comprised of a soft silicon insert which in turn contained silk hydrogels as a controlled-release substrate and drug carrier. The fabrication of the device has been reported elsewhere (Golding et al., (2016) PLoS One 11, e0155618). In brief, the outer cylindrical silicon sleeve (20-mm H x 18-mm D) were fabricated by casting silicon elastomer (Dragon skin 10, Smooth-on, Macungie, PA) against a 3D-printed mold which was designed using CAD software (Solidworks, Waltham, MA, USA) and printed using a Formlab 3D printer (Somerville, MA, USA).

The adjustable biodome was comprised of an acrylic tube (#8532K13, Mcmaster-Carr, Elmhurst, IL) cut in 1-cm long cylindrical tube was used as the body of the apparatus 100. Thread adapters were designed using 3-D CAD software (Inventor Professional, Autodesk, San Rafael, CA) and printed using a stereolithography 3-D printer (Form2, Formlab, Somerville, MA). The adapters were glued to the acrylic tube using a medical grade super glue. 2-ml HPLC vial caps (Agilent, (Santa Clara, CA). The PTFE/silicone septum was punched with a 3-mm biopsy punch to generate an access hole for the animal limb. To provide more room for the animal's leg to pass through the hole, the septum was cut along the central hole at four locations, a quarter-circle away from each other. Custom washers were either made from PDMS using soft-lithography or 3D-printed using the 3D printer. The cylindrical wall of the apparatus 100 insert was fabricated from a transparent polyester membrane filter (0.45 µm pore size, 12-µm thick, # 1300016, Sterlitech, Kent, WA). The filter was cut in rectangles (7 mm x 5 mm) and rolled around a metal rod of 1.5-mm diameter (#8907K62, McMaster). Then the wall was glued to a silicone bottom part using a silicone adhesive (Dragon Skin 10 FAST, Smoothon, Macungie, PA) to complete the insert.

The protective cage was assembled from threaded caps, a transparent acrylic body, and adapters. A donut-shaped septum that only allows one-direction bending was provided to prevent the detachment of the apparatus due to animal movement and tampering. The custom washer together with the bottom cap provide tunable pressure sufficient to hold the scaffold insert tightly against the wound bed, as well as keeping its position stable over a long-term experiment. It also comes with an access hole for media exchange. The apparatus insert contains a membranous side wall and a silicone bottom. The side wall can hold liquid necessary for keep the tissue moist as well as facilitating the gas exchange the silicone bottom serves as a septum for insertion of needle for media exchange.

### Device Removal and Maintenance

After 24h, animals were anesthetized and treated with an analgesic as previously described. The devices were then removed by cutting the single suture on either side of the leg, and the frogs were placed back into tanks containing a fungicide (Kordon methylene blue at concentrations of 1 mL /10 L frog water. After another 24 hr, the water was replaced with fresh 100% frog water. Once their devices were removed, animals were maintained for 18 months in frog water that was changed daily. Endpoint euthanasia was carried out by full-body immersion in frog water with 0.2% benzocaine. Regenerates, contralateral limbs, and brain tissues were collected and processed for histological analysis.

### Silk Processing

Silk fibroin solution was prepared by cutting and degumming 5 g of silkworm (*Bombyx mori*) cocoons (Tajima Shoji, Yokohama, Japan) in a solution of 0.02 M sodium carbonate (Na₂CO₃) for 45 min to remove non-essential protein substrates (*i.e.,* sericin). Fibers were washed in deionized (DI) water several times to remove the Na₂CO₃ and then dried inside a fume hood overnight at 22°C. Dry silk fibers were then dissolved in a 20% (w/v) solution of 9.3 M lithium bromide (Sigma-Aldrich, St. Louis, MO) and placed in an oven set to 60°C for 4 h. The solution was then dialyzed in DI water using a dialysis cassette (molecular weight cut-off of 3.5 kDa, Thermo Fisher Scientific, Waltham, MA) with gentle stirring. Water was changed six times over a 48-h period. The dialyzed solution was centrifuged three times at 13,000 g, 4°C for 20 min each followed by filtering through a cell strainer (40-µm pore size, Thermo Fisher) to remove impurities. To determine the concentration of the filtered solution, a 0.5 ml sample was dried completely overnight in an oven. Having evaporated the water content, the dried silk was weighed and the concentration in % (wt/v) was calculated as the ratio of the weight of the dried silk over its initial volume of 0.5 ml.

Silk hydrogels were formed by cross-linking liquid silk fibroin. A 45-mb silk 3%w/v) and horseradish peroxidase (HRP) solution (20 U/ml was cast into a 24-well plate and incubated at 37°C for 45 min to complete the gelation. Gel compression strength and moduli of the gels was tested according to the method of Golding et al. (2016), PLoS One 11, e0155618.

### Scaffolds with Aligned Pores

Silk scaffolds with aligned pores were fabricated using 5 µl of the 4% (wt/v) silk solution. The solution was placed on top of an aluminum plate and a steep temperature gradient was induced by merging plate in liquid nitrogen (LN2). Finger-like columns of ice crystals growing from the cold surface created channel-like structures internally inside the silk solution as it solidified. After 10 min of cooling, the frozen solution was lyophilized for over 24 h to remove the water. The sponges were trimmed to fit in the Biodome as an insert and were sterilized in ethylene oxide and stored at room temperature in sterile condition until use.

Collagen scaffolds with aligned channel-like porous structure were fabricated by controlled directional freezing and freeze-drying of a 1.5% (wt/wt) collagen solution (in a similar fashion as for the silk scaffolds). The scaffolds were cut in a cylindrical shape (4-mm long and 1.5-mm diameter) and placed inside the biodome insert using forceps.

### Material Characterization

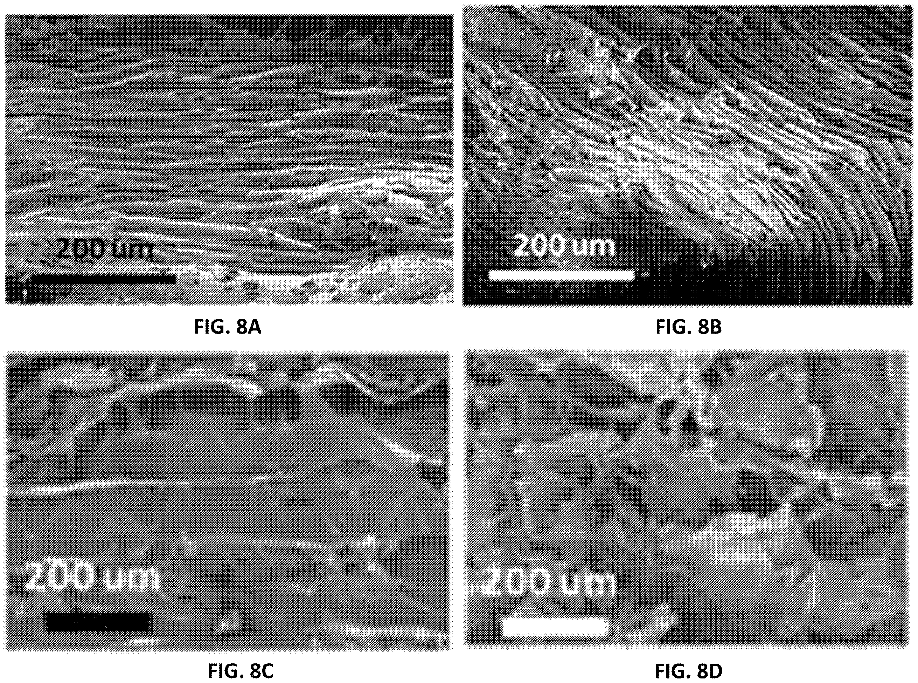

Morphology of the scaffolds was characterized using scanning electron microscopy (SEM) and fluorescent light microscopy. For SEM imaging, scaffolds were cut in half using a razor blade to expose the internal geometry of the pore. Prior to imaging, the scaffolds were sputter coated with gold to increase the conductivity. The SEM imaging was conducted on a microscope (Zeiss EVO MA10) set at 5kV. For fluorescence imaging, scaffolds were stained with 2 µg/ml fluorescein isothiocyanate (FITC) in PBS and imaged using a Keyence microscope (BZ-X800, Keyence, Japan). Compressive stiffness and elastic modulus of the scaffolds was determined using an Instron Testing System. FIG. 8A is a silk scaffold with channel-like pores aligned along the long axis. FIG. 8B is a silk scaffold with pores aligned perpendicular to the long axis. FIG. 8C is a SEM image of collagen scaffold with pores aligned along the long axis. FIG. 8D is a collagen scaffold with pores aligned perpendicular to the long axis.

### Therapeutic Composition

The hydrogels were prepared with a final concentration of 3% (w/v) silk solution, horseradish peroxidase (HRP) of 20 U/ml, and hydrogen peroxide (H2O2) of 0.01% wt/v. The liquid solution was poured into the silicon sleeve and allowed to gelate for 30 min prior to attachment to the limb stump of the animal. For the cocktail-loaded devices, 0.014 µg/ml of 1,4 (dihydrophenonthrolin-4-one-3carboxylic acid) DPCA (Catalog # 71220, Caymen Chemicals, MI, USA), 0.5 µg/ml of brain derived neurotrophic factor (BDNF) (Catalog # 450-02, Peprotech, MA, USA), 0.5 µg/ml of growth hormone (GH) (Catalog # 100-40, Peprotech, MA, USA), 0.036 µg/ml of resolvin D5 (Catalog # 10007280, Caymen Chemicals, MI, USA), 0.015 µg/ml of retinoic acid (Catalog # 11017, Caymen Chemicals, MI, USA) were loaded into the liquid silk solution prior to insertion and gelation into the silicon sleeve.

### In Vitro Release Studies.

To determine the release profiles of the drugs used in the present example, 50 µL of each hydrogel solution loaded with specific amounts of the specified drugs were added into 1.5-ml microcentrifuge tubes and incubated for 45 min at 37°C to a complete gelation. Then, 1 ml of Dulbecco's phosphate buffered saline (DPBS 1X, Gibco) was added into each vial followed by incubation at 37°C. At fixed time points, 300 µl of supernatant was collected for analysis. Release solutions of drug-free silk hydrogels were used as controls. Standard curves were determined by measuring the optical density of solutions with known concentrations. All the release experiments were carried out in triplicate to ensure accuracy. For RA and 1,4-DPCA, optical density of the release solutions was determined on a UV-transparent 96-well plate (Corning, Corning, NY) using a microplate reader SpectraMax M2 (Molecular Devices, San Jose, CA) operated by SoftMax Pro 6 software. Detection was performed at a wavelength of 280 nm and 350 nm for 1,4-DPCA and RA solutions, respectively.

The concentrations of BDNF and GH in the release samples were determined using Enzyme-Linked Immunosorbent Assay (ELISA) kits containing monoclonal antibodies designed for BDNF and GH (#BGK23560 and # BGK01241, Peprotech, Rocky Hill, NJ, USA). Sample preparation and measurement were performed according to the manufacturer's protocol. Optical density of the prepared samples was read at 450 nm using the SpectraMax M2 plate reader. Release samples of Resolvin D5 was filtered with a protein filtration column (MWCO = 3 kDa, #UFC500324, Fisher) to remove the high-molecular weight fibroin content. Then optical density of the samples was determined at a wavelength of 244 nm using the SpectraMax M2 plate reader.

### Soft Tissue Imaging.

At regular intervals over the 18-month maintenance period, animals were evaluated for soft tissue repatterning and bone regrowth. Animals were anesthetized as described previously and high-resolution images of their wound sites and regenerate dimensions were captured using a DSLR camera (Canon EOS Rebel T7i). To ensure replicability, the amputation plane served as a standard reference point for all measurements. The site of amputation was easily identified due a reliable tapering of the limb at the point of incision. Each measurement consisted of a linear assessment of length between the amputation site and the most distal end of the regenerate.

### In Vivo, X-Ray and Micro CT Bone Imaging

In addition to soft tissue measurements, bone length was assessed using a handheld x-ray device (Nomad Pro 2TM) using standard imaging settings of 60 kV, 2.5 mA for 0.20 s exposure time, according to the imaging protocols of Golding et al. (2016) PLoS One 11, e0155618, and Herrera-Rincon, et al., (2018) Cell Rep 25, 1593-+. Every animal was subjected to the same dose (0.12 mSv) at fixed time points. Computerized tomography (CT) in a viva CT 40 scanner (Scanco Medical, Switzerland) was performed to visualize the detailed microarchitecture of bone at the end of the 18-month regeneration period following euthanasia by benzocaine overdose (0.2% fully-body immersion). Distal trabecular bone and midshaft cortical bone sections (615 slices/animal, 76µm/slice, integration time of 300 ms) were visualized and rendered into 3-D images for further quantification. The radiation dose was by established manufacturer guidelines using local CT Dose Index (CTDI) which ranged from 453 mGy to 1255 mGy.

### Histology and Immunohistochemistry

To characterize the effect of treatment on post-amputation limb re-growth and re-patterning, histological analyses were carried out on regenerates and contralateral limbs at fixed intervals over time. Tissues were collected at 18 mpa. Long-term regenerate tissues were fixed overnight in 4% paraformaldehyde (PFA) in PBS and decalcified for 2 weeks by exposure to increasing concentrations (10%-15%) of ethylenediaminetetraacetic acid (EDTA) (pH7.4). Once decalcified (confirmed using x-ray), tissues were gradually equilibrated to 30% sucrose before embedding in OCT (Sakura FInetek,USA). Samples were frozen in liquid nitrogen. Limb tissues were serially sectioned at 14 µm using a cryostat (Leica CM1850) and placed on glass slides. Cross sections were taken at 14um intervals across the limb from the tibiofibular region above the original amputation site to the patterned region at the terminus of the limb. In order to visualize the patterning, the end portion of the limb was sectioned horizontally at 14um intervals. Sections were dried for at least an hour before storage at -80°C.

For immunohistochemistry, slides were equilibrated to room temperature for at least 2 hr prior to staining. Slides were post fixed for 5 min in 4% PFA and then blocked in blocking buffer (PBS with 0.1% Triton X-100 and 10% normal goat serum) for 1 hr. Primary antibodies against acetylated α-tubulin (1:100), TGF-β (1:250), smooth muscle actin (1:100), laminin (1:100), fibronectin (1:500), and phosphohistone H3 (1:250) were used. Slides were stained individually with each antibody except anti-smooth muscle actin and anti-laminin, which were stained together. Primary antibodies were incubated on the slides overnight. After washing in PBS, alexa-fluor secondaries (1:500, ThermoFisher Scientific) were applied in blocking buffer for 2 hr. Slides were again washed in PBS and stained with DAPI 1:200 in PBS for 20 mins. Slides were mounted in Fluoromount-G (ThermoFisher Scientific) and cured for at least 24 hr before imaging.

Sections were imaged using an EVOS FL automated imaging system (ThermoFisher Scientific). Entire sections were collected and stitched together for analysis.

### Immunostained Section Analysis

All statistical analyses were performed in IBM SPSS version 20 software. Assumptions of normality were tested before the use of parametric testing including ANOVAs, t-tests, and correlational analyses (Pearson's r). Non-parametric analyses, Mann Whitney Wilcoxon Test or Kruskal-Wallis test were conducted for comparisons where the data was not normally distributed. Significant differences were assumed if p values were below a threshold of 0.05 (two-tailed hypothesis testing).

### Assessment of Sensorimotor Thresholds

To evaluate the sensorimotor capability of the regenerate, animals were assessed at 18 months post-amputation. Each animal was placed into a glass tank filled with 2 L of frog water and acclimated for 5 mins until movement ceased completely. A video camera (iPod Touch 5th generation, Apple, CA, USA) was placed over the enclosure to capture recordings of the testing procedure. Standardized Von Fry Filaments (Touch Test, Stoelting, IL, USA) were used to assess the sensory threshold of the regenerates. Filaments ranging from 0.008 g to 300 g in force were applied to the distal portion of the regenerate, from lowest force to highest. The first filament that induced a clear response (movement from the stationary position) was recorded. Animals were tested twice over a 2-day period and the average threshold was reported.

### Statistical Analysis

All statistical analyses were performed using IMB SPSS v20. First, data were tested for homoscedasticity by Levene's test. Unifactorial analysis were performed on normally distributed data by unpaired, two-tailed student's t-test (two independent groups), or one-way ANOVA test (multiple independent groups) followed by post-hoc Scheffe's test (when P <0.05). When variable "time" was considered, a bifactorial analysis was performed by two-way ANOVA. Statistically significant differences between treatment groups (No device, Biodome only and cocktail treatment) at each specific time point were determined by using student's t-test. In non-normal distributed data, a Kruskal-Wallis test followed by pot-hoc Dunn test (when P < 0.05), respectively, was conducted. The significance level was set to 0.05 in all cases. The statistical values are reported as means ± standard deviation or means ± standard error of the mean, where indicated. When appropriate, dot or scatter plots are used for highlighting the individual variability within each experimental group.

### RNA Extraction

Following amputation, device attachment and removal, and 24-hours of treatment, regenerate tissues were harvested at 11-, 24-, and 72-hr post-amputation for next generation sequencing (NGS). Samples consisted of 1 cm thick tissue blocks from the distal wound site. Brains were also collected and flash frozen. Tissue was extracted using TRIzol (ThermoFisher Scientific) as per the manufacturer's protocol and total RNA quality and quantity was assessed using a Nanodrop Spectrophotometer (ThermoFisher Scientific).

### Next Generation Sequencing (NGS)

1.1µg of total RNA was sent to the Tufts Genomic Core. RNA quality was assessed via bioanalyzer and high-quality RNA was used for library prep with the TruSeq stranded RNA Library Prep Kit with RiboZero Gold (Invitrogen). Libraries were then multiplexed and single end, 50nt sequencing was performed on the Illumina HiSeq 2500. Raw read files were sent to the Bioinformatics and Biostatistics core at Joslin Diabetes Center.

### NGS Analysis

The reference genome for *Xenopus laevis* was downloaded from the NCBI Genome database, assembly GCA_001663975.1. Reads were aligned using STAR aligner (Dobin et al., Bioinform. (2013);29(1):15-21. doi: 10.1093/bioinformatics/bts635. Epub Oct 25. PubMed PMID: 23104886; PubMed Central PMCID: PMC3530905.) and aligned reads were counted using featureCounts (Liao et al., (2014) Bioinform. 30(7):923-30). Genes with expression counts more than 1 count per million (cpm) in at least 3 samples were included in the analysis, and counts were normalized by weighted trimmed mean of M-values (TMM, Robinson et al., (2010) Genome Biol. 11, R25.). Voom transformation was performed (Law et al., Genome Biol. 15, R29.) to transform counts into logCPM, where CPM = 1e+6 * count of a gene / (total counts of the sample * normalization factor of the sample). Voom transformation also estimates the mean-variance relationship and use it to compute appropriate observational-level weights, so that more read depth gives more weights. To further down-weight the outliers, sample-specific quality weights (Ritchie et al., (2006) BMC Bioinform. 7, 261.) were collected and combined with the observational-level weights.

Differentially expressed genes were identified using limma (Ritchie et al.,, Nucl. Acids Res. 43, e47.). Moderated t-tests were performed to detect genes that were differentially expressed between two groups. Genes with FDR<0.25 were considered significantly changed.

Gene sets for pathway analysis were obtained from MSigDB Collections and gene sets that belong to canonical pathways (CP) or gene ontology (GO) were selected. Analysis was performed with the Fry function in the Rotation Gene Set Test (Roast) in the limma R package (Wu et al., (2010) Bioinform. 26, 2176-2182.). Gene sets that are coordinately up, coordinately down, and mixed all were considered significant if P < 0.05 and FDR < 0.05.

### Network Analysis

Gene modules were identified by co-expression analysis in CeMiTool in R (Russo *et al.,* 2018) based on the logCPM values for control and cocktail-treated groups at 11 hours, 24 hours, and 7 days after limb amputation. In CeMiTool, a variance stabilizing transform was applied to remove the dependence between mean and variance parameters and genes were filtered based on expression levels at a threshold of p < 0.1. Modules of co-expressing genes were identified within the dataset based on an automatically generated scaling value beta (β=10) and a minimum module size of 30 genes. To assess how the enrichment of these modules varied over time and group, module enrichment was calculated based on sample annotation. To determine which biological functions are associated with each module, over representation analysis was performed using a pathway database from Reactome and pathways were considered to be significant for p < 0.05. Annotated module graphs combined gene-gene interaction data from Kyoto Encyclopedia of Genes and Genomes (KEGG) and Chemical and Genetic Perturbations (CGP) to map the interacting genes contained in each module.

### qPCR Methods

The same total RNA submitted for RNAseq analysis was DNase treated using RQ1 RNase-free DNase kit (Promega Corp., Madison, WI, USA). The resulting RNA (0.5 µg) underwent a second DNase treatment followed by cDNA synthesis using the Verso cDNA Synthesis Kit (ThermoFisher Scientific). Quantitative analysis of the amount of gene product using the Step One Plus Real Time System (Applied Biosystems, USA). Each 10 µl reaction was run in duplicate and contained: 5 µl of 2x PowerUp SYBR Green Master Mix (Applied Biosystems, USA), 0.5 µl of 10 µM of forward and reverse gene specific primers, and 1.33 µl of diluted cDNA template. The relative expression was analyzed using the delta-delta Ct method with the average of all ND expression across timepoints used as calibrator for all samples.

### Results

### 1. Induction of Leg Regeneration by Multi-Drug Regenerative Treatment

Adult Xenopus hindlimbs were amputated at the mid-point along the tibia and fibula, and fitted with biodome devices that either contained a silk-based hydrogel with a 5-drug multi-drug treatment (MDT) or hydrogel only. Control animals were amputated without treatment. After a 24-hr exposure to the BioDome, devices were removed, and animals were maintained for up to 18 months with periodic assessment of regeneration and re-patterning of the hindlimb regenerate. The protracted observational window was selected based upon Alibardi's (2018) calculations that predict about 1.5 years are needed to regenerate an anuran limb based on projected modeling using newt stump diameters.

X-ray images were acquired to measure limb length as a function of months post amputation. X-rays illustrated that regenerates associated with both the biodome only and multi-drug treatment (MDT) condition were longer than control (no device) regenerates after 4 months, an effect that disappeared after 4 months and then reappeared at 8 months and persisted over time. (two-way ANOVA, between-subject factor treatment exposure, within-subject factor regeneration time F(2,19) = 61.9, p < .05). Limb length relative to the amputation site was greater for the MDT group relative to other group as early as 0.5 months post amputation (mpa) and continuing to 4mpa (p<0.05), suggesting that the MDT increased early growth rate. In months 6-8, growth slowed significantly and hindlimb regenerates associated with the Biodome only group achieved comparable lengths relative to MDT-exposed animals (p>0.05). A secondary increase in late stage growth after 9 mpa shows that the MDT group again displayed longer limbs relative to other groups, an effect that persisted until the final measurement at 18 mpa (p<0.01). Thus, the MDT group not only shows an ultimate increase in leg length compared to other treatment groups, but it also shows a secondary period of growth that is absent in the other treatment groups, particularly the no device group. These data suggest that the brief exposure to the MDT and BioDome facilitates longer leg regrowth.

Not only was leg length increased, but 76% of animals exposed to MDT also displayed thicker, more complex regenerate morphologies relative to the featureless, heavily pigmented spikes of the no device group. Specifically, the distal segments of hindlimb regenerates associated with the MDT group presented with flattened, paddle-like structures with projecting buds characteristic of digits. In contrast, no device and biodome only conditions were exclusively associated with featureless regenerate spikes, the provided MDT condition reliably produced patterned, paddle-like morphology with the presence of distal buds. The BioDome group had intermediate phenotypes, with 20% of BioDome animals displaying thicker cartilaginous spike and hook-like distal projections but limited patterning. No animals in the no device group displayed no unique phenotypes.

### 2. Induced Legs Exhibit Sensory Function

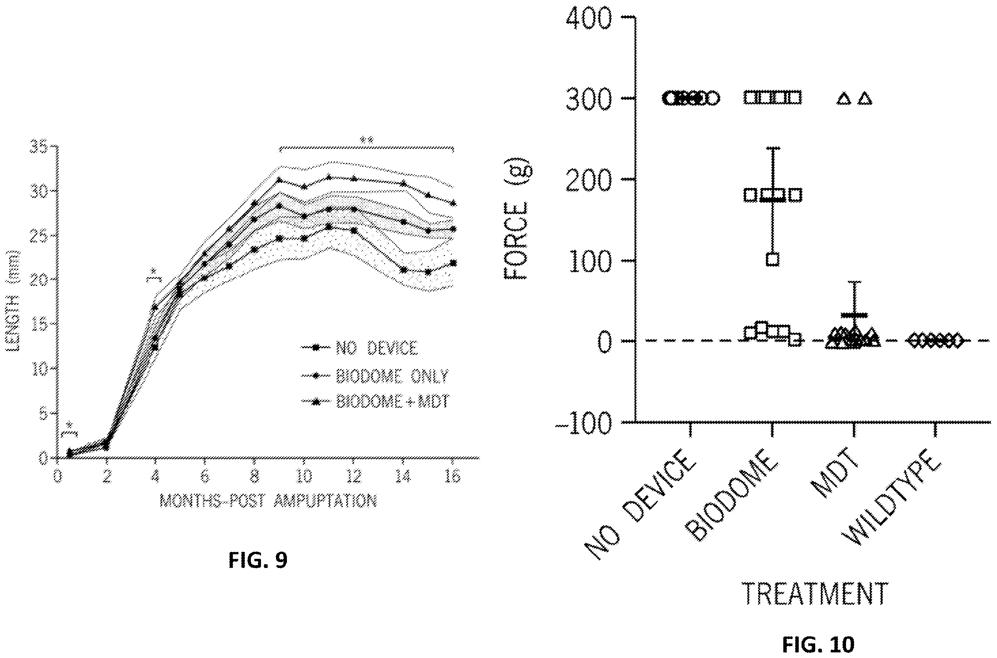

Next, to assess whether the 18 mpa regenerates had regained sensory function, a sensorimotor assessment was conducted. Using standardized Von Frey (VF) filaments (minimum force: 0.008g; maximum force: 300 g), the regenerated right hindlimb was probed at the most distal end with filaments of increasing strength until the maximum force was applied. The first filament that induced a clear response (movement from the stationary position) was recorded, and behavioral assays were averaged over 2 d. MDT-treated hindlimb regenerates displayed comparable stimulus-response patterns to the non-amputated group (p > 0.01; FIG. 10), indicating significant reinnervation and neuromuscular re-integration. In the biodome group, responses varied markedly between individual animals, with a group performing at near-normal levels and a group not able to detect the stimulus (56.6 ± 98.8 g), while untreated animals reliably failed to display any response to applications of force at any value up to and including 300 g. Thus, we conclude that the MDT treatment facilitated sensorimotor integration similar to that of an unamputated limb. Interestingly, the biodome alone group also was sometimes able to facilitate regrowth of a limb with an intact sensory system, indicating that hydration and structural support may be important to maintain innervation and facilitate nerve regrowth.

### 3. MDT Exposure Increased Hindlimb Bone Length and Complexity

The internal structure of the regenerates was characterized using microCT and X-ray images. As overall hindlimb length was significantly improved by the treatment (FIG. 9), a study of the bone component of the regenerate was subsequently studied. The microCT and X-ray images of regenerates reveal increased bone volume and length associated with the provided MDT compositions relative to biodome only and controls.

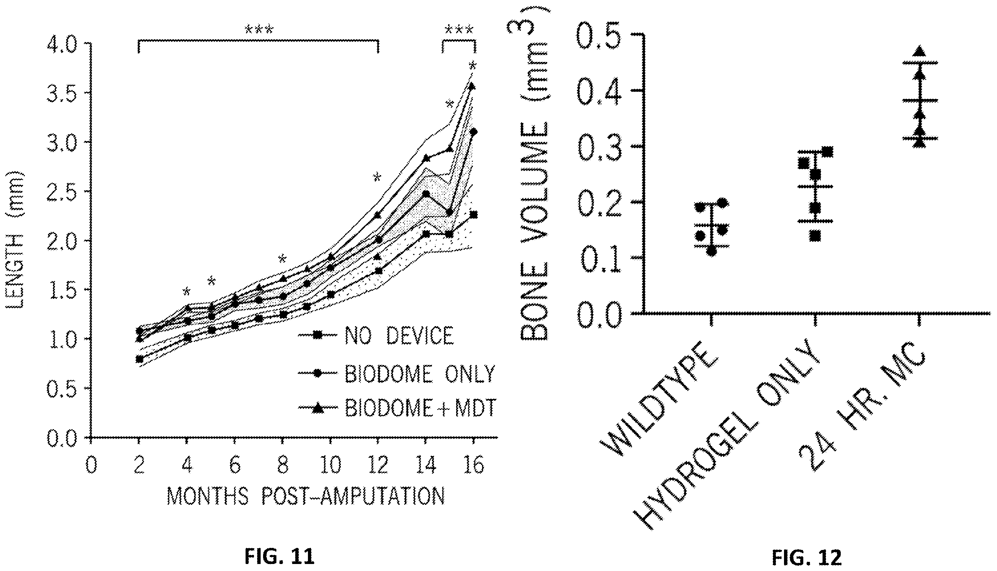

X-ray images confirmed the presence of complex morphology and increased bone length in MDT regenerates relative to biodome only and no device control experiments. Beginning with measurements at 2.5 mpa, dense tissue projecting outward from the amputation site was observed in the MDT and biodome groups, but not the no device controls. The MDT group also displayed dense, segmented bone fragments at the distal end of the regenerates. As predicted, the MDT condition displayed increased bone length relative to other conditions beginning at 4 mpa (p < 0.05) with an inflection at approximately 8 mpa (similar to soft tissue measurements). As shown in FIG. 11, bone length differences between the MDT and Biodome group were periodically observed, but the difference in length between those groups and no device controls were maintained throughout (p < 0.05).

To gain a better understanding of the microarchitecture of the regenerated bone, MicroCT was performed at 18 mpa. MicroCT imaging allowed for 3-dimensional rendering of underlying bone to be visualized and measurements of bone volume to be taken without disturbing external soft tissue. MicroCT data confirmed the presence of significant bone fragmentation in the distal region of MDT hindlimbs. Notably, fragmentation does not occur at the amputation site but instead emerges spontaneously after a period of growth at a point along the bone that is equivalent to the contralateral joint. These repatterned segmentations were common within the MDT group. As shown in FIG. 12, bone volume as measured through microCT data was greater for the hindlimb regenerates of the MDT group relative to other groups. Volumetric quantification confirmed increased growth in provided MDT regenerates (One way ANOVA (F(2,15)=11.15, p<0.001)). Tomograms also revealed marked similarity between unamputated and MDT-treated bone anatomy. Repatterning was evident in the provided MDT regenerates including the re-expression of bone features that are normally associated with the attachment of musculature. Specifically, at 17 mpa, the MDT group displayed bone features characteristic of muscular attachment as well as the presence of joint segmentation. These data indicate that the MDT condition is associated with significant remodeling of bone anatomy, consistent with active processes re-shaping bones and ultimately achieving a morphological complexity of features approximating the pre-amputated limb.

### 4. Complexity of Regenerated Limbs

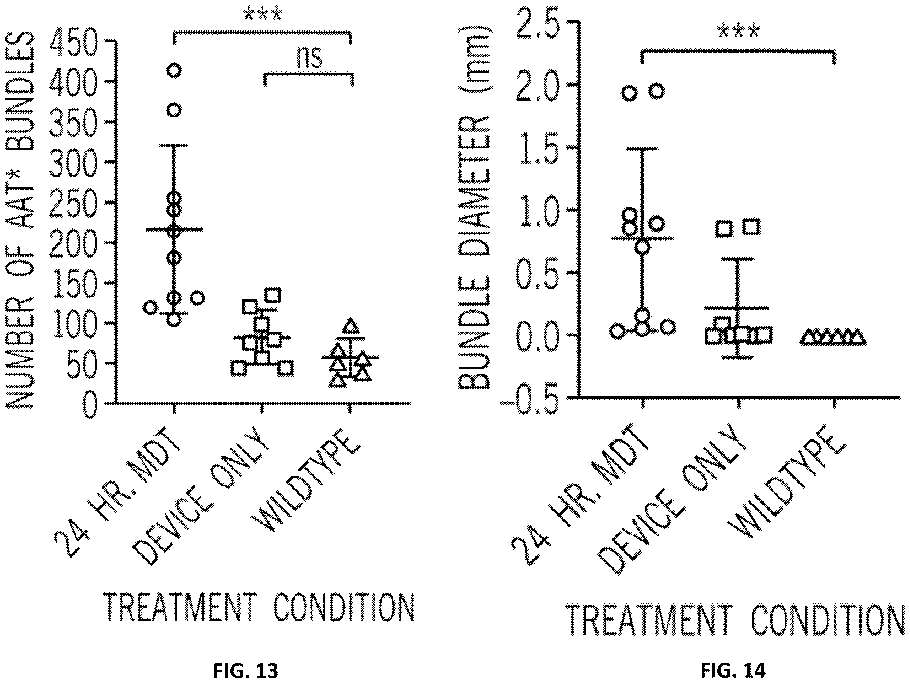

While MDT-treated animals did show increased bone growth, molecular changes at the cellular level in the regenerated limbs was also assessed. Immunohistochemistry was performed to assess tissue architecture associated with regeneration and re-patterning after 18 mpa. As shown in FIG. 13, reinnervation as measured by acetylated α-tubulin (AAT) staining revealed a significant increase in the number of AAT+ nerve bundles associated with the 24 hr MDT condition relative to the no device group (U=13, P=0.0014). The number of AAT bundles were comparable when comparing the no device and biodome only group, indicating that the MDT treatment influenced nerve bundle regrowth and innervation of the regenerate. Not only were there more bundles, but the 24 hr MDT group also exhibited significantly larger AAT bundle diameters relative to the no device group, as shown in FIG. 14. There was no significant increase in bundle size between the biodome only and no device groups.

Kruskal-Wallis test showed that the number of AAT positive bundles at 18 mpa were significantly different across conditions, H(15.12) = p <0.005. Mann-Whitney U post-hoc analyses revealed the major source of variance was an increase of AAT bundles associated with the 24 hr MDT condition relative to the uninjured animals, U = 13, P = 0.0014. The number of AAT bundles were comparable when comparing uninjured animals and the biodome only group (p=0.1812). Next, we examined ATT bundle dimensions, revealing significant differences across conditions, H(11.74) = p= 0.0028. The 24hr MDT group exhibited greater ATT bundle diameters relative to uninjured animals. Again, there were no significant identified when comparing the biodome only and uninjured animal groups.

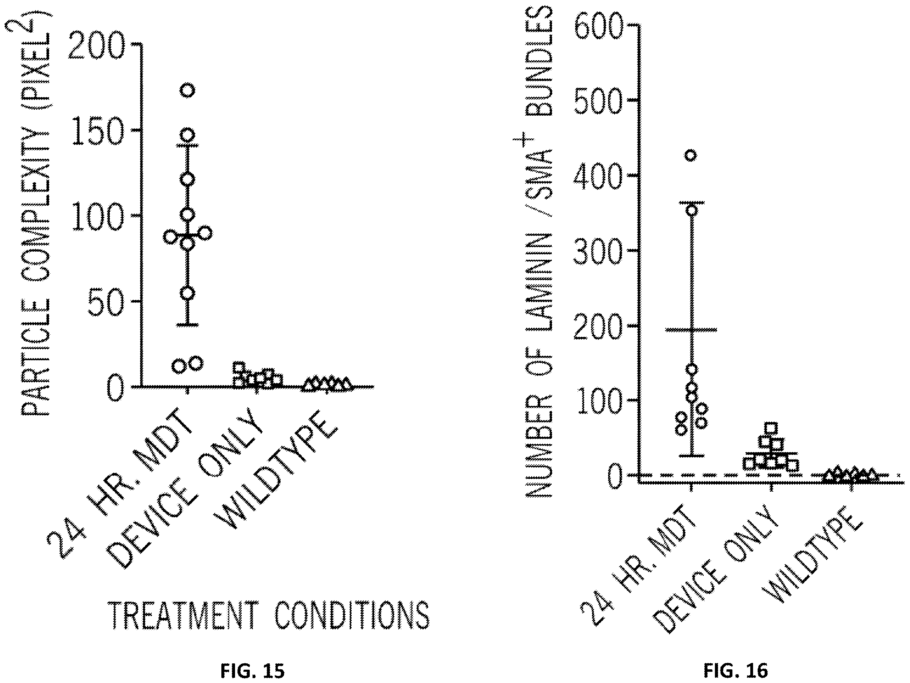

In order to assess changes to connective tissue architecture, we assessed Fibronectin expression pattern after 18 mpa. As shown in FIG. 15, increased particle complexity in the MDT-treated regenerate reflected the degree of structural complexity observed in the MDT condition. Specifically, fluorescent images revealed an increased nerve regeneration and extracellular matrix re-patterning in regenerate 18 months after initial amputation and exposure to multi-drug treatment. Consistent with other results, the 24 hr MDT group displayed increased particle complexity relative to both the biodome only and no device groups, indicating that the MDT itself likely facilitates elaboration of cartilage and connective tissue in the regenerate.

In order to assess vascularization of the regenerate, laminin and smooth muscle actin (SMA) expression at 18 mpa was compared across conditions, as shown in FIG. 16. Fluorescent images of cross sections of the regenerates obtained 18 months post-amputation were double-stained for two markers of vascularization, one associated with smooth-muscle (SMA-red), and the other for the basement membrane (green). The comparison of the sections revealed a marked increase in SMA/Laminin positive bundles in the multi-drug treatment group, as compared to the device only or untreated groups. (H(19.84) = p < 0.001). The number of blood vessels identified in each section was significantly larger in the MDT-treated group as compared to both the biodome only and no device groups (H(19.84) = p<0.001), indicating that the MDT facilitates vascularization of the regenerate, more than doubling the number of blood vessels in the MDT-treated regenerate vs the no device condition.

### 5. Wound Closure, Increased Sox2 Expression, and Re-Epithelization

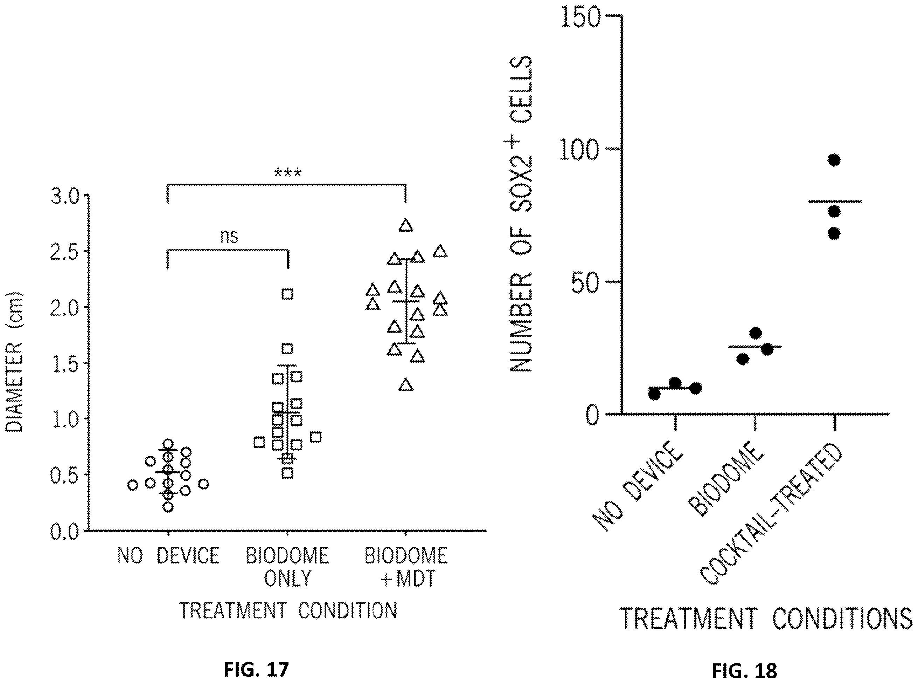

Considering the remarkable long-term outcomes, the effect of the MDT at early time points after amputation was assessed during the early phase of this process. We first noticed that wound closure at 0.5 mpa was significantly reduced in the MDT group as compared to no device animals (p<0.05; FIG. 17). MDT-exposed animals displayed an average wound diameter of 2.02 cm ± 0.40 cm, significantly larger than the biodome group (1.25 cm ± 0.41 cm) or the no device group (0.56 cm ± 0.17 cm). The delayed wound closure was predictive of successful re-patterning 18 mpa, with animals who had the largest wound site at 0.5mpa showing the most growth and repatterning at 18mpa.

Without being bound to a particular theory, it is contemplated that the larger wound site would provide a larger blastema, which might contribute more material to limb regrowth. As shown in FIG. 18, Blastema proliferation was assessed via immunohistochemistry for SOX2, a proliferative cell marker. There were significantly more SOX2-expressing cells in the MDT group relative to other groups (p < 0.05), indicating that there is more proliferative tissue to generate a new limb. Finally, soft tissue at 2.5 months was similarly predictive, with thicker tissue and increased bone length in the MDT hindlimbs compared to control. Fluorescent images obtained 2.5 mpa revealed that 24 hour exposure to MDT compositions increases stemness, inhibition of wound formation resulting in longer regenerates in early regeneration processes. Increased soft tissue growth and re-epithelization was also observed 2.5 months after amputation in the MDT-exposed group, which mirrored the increased length of bone tissues as confirmed by x-ray imaging.

### 6. Transcriptomic Analysis of Regenerative Induction

To gain a more careful understanding of the gene expression changes in response to the acute exposure to the MDT treatment, transcriptional mechanisms downstream of the intervention were characterized. RNA-sequencing (RNASeq) was performed comparing the transcriptome of the blastema that was obtained from MDT vs. untreated animals at 11 hr, 24 hr, and 7 d post- amputation. A heatmap comparing gene expression levels of MDT animals compared to a no device treatment shows significant differential gene expression at 11 HPA that persists to 24 HPA. By 7 DPA, however, dynamic gene expression levels return to normal, indicating a period of dynamic gene expression within 24 hours of amputation.

The number of differentially expressed genes was determined after multiple testing corrected the p value. The Q value was set at a false discovery rate (FDR) of 0.05, and differentially expressed genes were considered to be those transcripts passing this FDR and those showing a log2 fold change of 2. When comparing the blastema of MDT animals to the untreated animals there was a large dynamic change in expression profile over the 7 d after amputation. The same genes that were over or under expressed experiences 24 hr after a switch in activity after 7 d. To narrow in on the drastically altered expression of these genes, the FDR was set to log3 or a fold change of 3, and the top 15 differentially expressed genes were compared between groups. When comparing the MDT-treated to wildtype blastema tissue it was found that the top 15 highly upregulated genes were related to neural regulation (e.g., Brain-specific kinases (BRSK), neuropeptide FF, D1C Dopamine receptor, neuroligin) brain with the highest expression 11 hr post-amputation. This expression level decreased 24 hr and 7 d post amputation (dpa). Wnt7a, a gene involved in the development of anterior-posterior axis, was also upregulated at 11 hr post-amputation (hpa) and subsequently increased by 7 d post-amputation. Conversely, the major genes that were downregulated were predominantly associated to muscle structure (myosin-4, microfibril-associated glycoprotein) and metabolism (e.g., sarcolipin). The pattern of these differently expressed genes between MDT- treated and control blastema was opposite to that of the upregulated genes, in that, the expression of the down regulated genes increased from 11 hpa to 7 dpa.

Highly regulated genes in the MDT animals were compared to the genes in no-device animals. Upregulated genes include nervous system-specific transcripts that suggest an important role of neuroprotective proteins just after amputation. Downregulated genes include metabolic and muscle-related transcripts, suggesting that resources are being directed away from muscle maintenance towards stabilization of the tissue. GO analysis of metabolic and biosynthetic pathways reveals an early downregulation (at 11HPA and 24HPA) that increases in rate by 7 dpa.. Table 1 illustrates gene expression levels of MDT animals compared to no device treatment for 11 hpa, 24 hpa, and 7 dpa..

**Table 1**

| Gene Name | 11 hpa | 24 hpa | 7 dpa |
|---|---|---|---|
| Neural proliferation differentiation and control protein 1-like | 4.43 | 3.76 | -0.777 |
| Brain specific kinase 146 L homeolog | 3.47 | 0 | -1.81 |
| Neuropeptide FF receptor 1 L homeolog | 3.37 | 2.59 | -0.421 |
| Metallophosphoesterase domain-containing protein 1 | 3.16 | 0.17 | -1.1 |
| MAM domain-containing glycosylphosphatidylinositol anchor protein 2-like | 3.07 | 0.57 | 0.903 |
| Neuroligin-1-like | 2.8 | 0.91 | -1.27 |
| Protein Wnt-7a-like | 2.79 | 2.46 | -1.5 |
| Oligodendrocyte lineage transcription factor 2 L homeolog | 2.79 | 3.58 | -0.419 |
| Brain-derived neurotrophic factor-like | 2.7 | 0 | 1.83 |
| D(1C) dopamine receptor S homolog | 2.47 | 1.84 | -0.516 |
| Hyperpolarization activated cyclic nucleotide gated potassium channel 1 L homeolog | 2.45 | 3.09 | -0.419 |
| Sarcolipin-like | -3.33 | -4.17 | -3.71 |
| Serine protease 33-like | 0.86 | -1.39 | -3.73 |
| Myosin-4-like | -0.866 | 0.656 | -3.81 |
| Tryptophan hydroxylase 1L homeolog | 0.855 | 1.01 | -3.94 |
| Alpha-tectorin-like | 1.05 | 1.56 | -4.19 |
| Microfibril-associated glycoprotein 4-like S homeolog | 0.589 | 2.91 | -4.21 |
| Myosin heavy chain, skeletal muscle-like | -4.26 | -3.76 | -4.31 |
| Myosin-4-like | -3.02 | -4.28 | -4.9 |
| Potassium/sodium hyperpolarization-activated cyclic nucleotide-gated channel 4-like | 1.91 | 3.81 | -4.95 |
| Chymotrypsin-like protease CTRL-1 | 1.84 | 1.85 | -4.97 |
| Keratin, type I cytoskeletal 12-like | -1.19 | -3.23 | -5.08 |
| Myosin-4-like | -0.104 | 0.0797 | -5.22 |
| Thyrotropin-releasing hormone L homeolog | 1.73 | 1.42 | -5.23 |
| Levitide S homeolog | 0.848 | 2 | -5.87 |

An enrichment analysis which identifies classes of genes showed notable differences in the profiles of biological processes between groups. The web-based version of CEMiTool (Co-Expression Modules Identification Tool) was used to identify covarying gene sets in the MDT (CT) and no device (ND) animals. Covarying gene sets of high- fold change were classified into modules (M1-M4).

Early after amputation (11 hpa), there were more genes involved in metabolic regulation that are different between MDT and control. In addition, there is more dynamic gene expression at this time point. This resolves later (7 dpa), and the significant changes between MDT and reorganizing tissues or resetting the cell function landscape.

To reveal the types of processes regulated by MDT-exposure, the enriched pathways were grouped considering "large-scale functions." Co-expression analysis identified 4 gene modules across the control and cocktail-treated groups at 11 hr, 24 hr, and 7 d after limb amputation. These modules represent a categorization of genes based on their shared expression levels and statistical significance. Module 1 contained 607 genes significantly represented (p < 0.00509) within extracellular matrix organization, collagen formation, collagen biosynthesis and modifying enzymes, and hemostasis pathways, which may be representative of tissue disruption after injury. Module 2 contained 142 genes that are significantly present (p < 0.00009) within pathways associated with cell junction organization, laminin interactions, cell-cell communication, apoptotic cleavage of cell adhesion proteins, and non-integrin membrane ECM interactions, which may be representative of cell-cell communication and adhesion. The 105 genes in Module 3 were significantly present (p < 0.01096) in muscle contraction, acetylcholine activity, and myogenesis pathways. Module 4 contained 54 genes that are significantly (p < 0.00142) over-represented within glucose metabolism, muscle contraction, gluconeogenesis, and glycolysis pathways. When Modules 2, 3, and 4 were significantly enriched (p < 0.00024) and upregulated in MDT-treated blastema across all time points, suggesting that frogs receiving the pro-regenerative cocktail have sustained upregulation of genes associated with cell communication, myogenesis, and glucose metabolism relative to biodome only and no device controls. In contrast, these modules were enriched (p < 0.012), but downregulated, in the group that did not receive a treatment device at all timepoints assessed. However, at the 7-day time point Module 2 was significantly upregulated (p = 0.00074) in the same untreated group, suggesting that there is little difference in the enrichment of cell-cell communication pathways between the treated and untreated groups 1 week after amputation. The ECM and collagen-enriched Module 1 upregulated (p < 0.011) in the untreated group and is downregulated (p < 0.00027) in untreated samples over all time points.

In general, M2, M3, and M4 were upregulated in the MDT condition vs the ND condition across all timepoints, peaking at 24HPA. The exception is M1, which was downregulated in the MDT condition most highly at 11HPA and decreasing to 7 DPA.

### 7. Cumulative Release of Drugs from the Provided MDT Compositions

In order to assess cumulative release for the multidrug treatment (MDT) in the device hydrogel, hydrogel loaded with each of the MDT drugs was suspended in 1x DPBS (ThermoFisher) and incubated for 25 min at 37°C. Supernatant was collected every 5 min, and the concentration of each drug was determined via a microplate reader. Each drug/hydrogel mixture exhibited similar release kinetics, releasing around 70% of the total drug concentration within 10 min and not releasing more than 80% of the total amount loaded (except for retinoic acid, which released all of the drug by 25 min).

Other features, objects, and advantages of the present disclosure are apparent in the detailed description that follows. It should be understood, however, that the detailed description, while indicating embodiments of the present disclosure, is given by way of illustration only, not limitation. Various changes and modifications within the scope of the disclosure will become apparent to those skilled in the art from the detailed description.

## Claims

1. An apparatus for stimulation of tissue regeneration at a site in tissue of a subject, the apparatus comprising:
an inner sleeve (216) including an end having a first opening sized to receive the site in the tissue for regeneration and an opposite engagement receiving end (220) having a second opening, the inner sleeve defining an internal chamber (222) that extends between the first opening and the second opening, the internal chamber being sized to receive the site in the tissue for regeneration;
a protein matrix (228) disposed in the internal chamber of the inner sleeve, the protein matrix comprising a porous scaffold having pores that form substantially aligned channels; and
filter media (230) enclosing the second opening on the engagement receiving end of the inner sleeve.

2. The apparatus of claim 1, wherein the substantially aligned channels are substantially parallel to a longitudinal axis of the inner sleeve orthogonal to the first and second openings.

3. The apparatus of claim 1 or 2, wherein the internal chamber comprises a reservoir of an aqueous solution or dispersing media between the filter media and the protein matrix.

4. The apparatus of any of claims 1-3, wherein the inner sleeve internal chamber further comprises a therapeutic composition which is deliverable to the wound and promotes tissue regeneration.

5. The apparatus of any of claims 1-4, wherein the internal chamber of the inner sleeve further comprises a therapeutic composition.

6. The apparatus of claim 5, wherein the therapeutic composition comprises:
a growth factor;
an inhibitor of prolyl hydroxylase domain, PHD, enzyme;
vitamin A or a derivative thereof; and
a lipid mediator.

7. The apparatus of claim 6, wherein the therapeutic composition comprises a growth factor selected from the group consisting of brain-derived neurotrophic factor, BDNF, nerve growth factor, NGF, neurotrophin-3, NT-3, neurotrophin-4, NT-4, glial-derived neurotrophic factor, GDNF, ciliary neurotrophic factor, CNTF, and leukemia inhibitor factor, LIF, and combinations thereof, and/or
an inhibitor of PHD enzyme selected from the group consisting of 4,4α-dihydro-4-oxo-1,10-phenanthroline-3-carboxylic acid, 1,4-DPCA, N-[(1,3-dicyclohexylhexahydro-2,4,6-trioxo-5-pyrimidinyl)carbonyl]-glycine, 6-Amino-1,3-dimethyl-5-[(2-pyridinylthio)acetyl]-2,4(1H,3H)-pyrimidinedione, 6-Amino-1,3-dimethyl-5-[[2-(2-pyridinyl)-4-quinolinyl]carbonyl]-2,4(1H,3H)-pyrimidinedione, N-[(4-hydroxy-1-methyl-7-phenoxy-3-isoquinolinyl)carbonyl]-glycine, iron chelators, and a combination thereof, and/or
a derivative of vitamin A selected from the group consisting of retinoic acid, retinol, retinyl carboxylates, tretinoin, tazarotene, and a combination thereof, and/or
a resolvin selected from the group consisting of resolvin 5, interleukin 6, IL-6, interleukin 4, IL-4, tumor necrosis factor-alpha, TNF-alpha, nuclear factor kappa-light-chain-enhancer of activated B cells, NF-kB, and a combination thereof, and/or
an agent that functions in proximo-distal positional information, wherein the agent is selected from the group consisting of bone morphogenetic protein 9, BMP9, nodal growth differentiation factor, activin, transforming growth factor-beta, TGF-β, fibroblast growth factor 8, FGF8, and a combination thereof, and/or
a peptide or protein hormone.

8. The apparatus of claim 7, wherein the peptide hormone is selected from the group consisting of growth hormone, GH, insulin-like growth factor-1, IGF-1, transforming growth factor-beta-1, TGFβ-1, epidermal growth factor, EGF, Granulocyte-colony stimulating factor, G-CSF, fibroblast growth factor, FGF, and a combination thereof.

9. The apparatus of any one of claims 6-8, wherein the growth factor is present at a dose within the therapeutic composition from 0.1 µg/ml to 1 µg/ml.

10. The apparatus of any one of claims 6-9, wherein the inhibitor of PHD enzyme is present at a dose within the therapeutic composition from 0.004 µg/ml to 0.024 µg/ml.

11. The apparatus of any one of claims 6-10, wherein the vitamin A or derivative thereof is present at a dose within the therapeutic composition from 0.03 µg/ml to 0.27 µg/ml.

12. The apparatus of any one of claims 6-11, wherein the lipid mediator is present at a dose within the therapeutic composition from 0.006 µg/ml to 0.054 µg/ml.

13. The apparatus of any one of claims 6-12, wherein the peptide or protein hormone is present at a dose within the therapeutic composition from 0.1 µg/ml to 1.0 µg/ml.

14. Use of the apparatus of any one of claims 1-13 for stimulation of regeneration of tissue in a mammal in need thereof.
